# EUROPÄISCHE PATENTSCHRIFT

(11) **EP 0 873 371 B1**
(45) Veröffentlichungstag und Bekanntmachung des Hinweises auf die Patenterteilung: **24.11.1999**
(21) Anmeldenummer: 97900557.6
(22) Anmeldetag: 03.01.1997
(51) Int. Cl.: C08G 73/00, B01D 61/14

(54) **VERFAHREN ZUR HERSTELLUNG VON WASSERLÖSLICHEN, AMINOGRUPPEN ENTHALTENDEN KONDENSATEN UND ADDITIONSPRODUKTEN UND IHRE VERWENDUNG**
METHOD OF PRODUCING WATER-SOLUBLE CONDENSATES AND ADDITION PRODUCTS CONTAINING AMINO GROUPS, AND USE OF SAID CONDENSATES AND ADDITION PRODUCTS
PROCEDE DE PRODUCTION DE CONDENSATS ET DE PRODUITS D'ADDITION RENFERMANT DES GROUPES AMINO HYDROSOLUBLES, ET LEUR UTILISATION

(30) Priorität: 08.01.1996 DE 19600366; 28.05.1996 DE 19621300
(43) Veröffentlichungstag der Anmeldung: 28.10.1998
(73) Patentinhaber: BASF AKTIENGESELLSCHAFT, 67056 Ludwigshafen (DE)
(72) Erfinder: STEUERLE, Ulrich, D-69124 Heidelberg (DE); MEIXNER, Hubert, D-67069 Ludwigshafen (DE); DYLLICK-BRENZINGER, Rainer, D-69469 Weinheim (DE); REUTHER, Wolfgang, D-69118 Heidelberg (DE); KANTER, Hartmut, D-67256 Weisenheim (DE); HETTCHE, Albert, D-67258 Hessheim (DE); WEISER, Jürgen, D-69198 Schriesheim (DE); SCHERR, Günter, D-67065 Ludwigshafen (DE)
(86) Internationale Anmeldenummer: EP9700009
(87) Internationale Veröffentlichungsnummer: WO9725367

(56) Entgegenhaltungen:
- US-A- 4 144 123
- US-A- 5 055 197
- CHEMICAL ABSTRACTS, vol. 104, no. 4, 27.Januar 1986 Columbus, Ohio, US; abstract no. 20660, XP002028700 & J.APPL.POLYM.SCI., Bd. 30, Nr. 10, 1985, Seiten 4099-4111, G.GUISE ET AL.: "The chemistry of a polyamide-epichlorhydrin resin used to shrink-resist wool" in der Anmeldung erwähnt

## Beschreibung

Die Erfindung betrifft ein Verfahren zur Herstellung von wasserlöslichen, Aminogruppen enthaltenden Kondensaten und Additionsprodukten sowie ihre Verwendung als Retentions-, Entwässerungsund Fixiermittel bei der Herstellung von Papier, als Promotor bei der Leimung von Papier mit Alkyldiketenen, als Flockungsmittel für Klärschlämme, als Haftvermittler bei der Herstellung von Verbundfolien, als Additiv in Haar- und Hautpflegemitteln und als Mittel zur Immobilisierung von anionischen Wirkstoffen.

Wasserlösliche Aminogruppen enthaltende Kondensate oder Additionsprodukte werden seit langem als Retentions-, Entwässerungs- und Fixiermittel bei der Herstellung von Papier eingesetzt. So ist beispielsweise aus der DE-B-1 771 814 ein Verfahren zur Erhöhung der Retention von Fasern, Füllstoffen und Pigmenten bei der Papierherstellung, zur Beschleunigung der Entwässerung von Papierstoffsuspensionen und zur Aufarbeitung von Papiermaschinenabwässern bekannt. Als Prozeßhilfsmittel werden hierbei Reaktionsprodukte eingesetzt, die durch Umsetzung von wasserlöslichen oder in Wasser dispergierbaren basischen Polyamiden mit Vernetzern hergestellt werden, die mindestens zwei funktionelle Gruppen enthalten, die mit den Aminogruppen der basischen Polyamide reagieren. Solche Vernetzer sind beispielsweise α,ω-Alkyldihalogenide, α,ω-Dihalogenether, Epichlorhydrin, ω-Halogencarbonsäurehalogenide, Bis-Glycidylether, Bis-Epoxide, Divinylether, Divinylsulfon und Methylenbisacrylamid.

Wasserlösliche Aminogruppen enthaltende Additionsprodukte sind beispielsweise Polyethylenimine, die üblicherweise durch polymerisieren von Ethylenimin in Gegenwart von Säuren, Lewis-Säuren oder Halogenalkanen hergestellt werden, vgl. US-A-2 182 306 und US-A-3 203 910. Polyethylenimine werden ebenfalls seit langem als Retentions-, Entwässerungs- und Fixiermittel bei der Herstellung von Papier eingesetzt.

Aus der US-A-4 144 123 (entspricht der DE-B-24 34 816) ist ein Verfahren zur Herstellung von stickstoffhaltigen Kondensationsprodukten bekannt, bei dem man Polyamidoamine mit Ethylenimin pfropft und die so erhältlichen Umsetzungsprodukte mit α,ω-Bis(chlorhydrin)ethern von Polyalkylenoxyden bei Temperaturen von 20 bis 100°C reagieren läßt, wobei man die Reaktion bis zur Bildung hochmolekularer, gerade noch wasserlöslicher Harze führt, die - gemessen bei 20°C in 20 gew.-%iger wäßriger Lösung - eine Viskosität von mehr als 300 mPas aufweisen. Die so erhältlichen Kondensationsprodukte werden ebenfalls als Retentions-, Flockungs- und Entwässerungsmittel bei der Papierherstellung verwendet.

Weitere Prozeßhilfsmittel, die für die Papierherstellung in Betracht kommen, sind Reaktionsprodukte von Polyalkylenpolyaminen oder Ethylendiamin mit beispielsweise Dichlorethan oder anderen mindestens bifunktionellen Vernetzern. Solche Reaktionsprodukte sind beispielsweise aus der EP-A-0 411 400 und der DE-A-2 162 567 bekannt.

Aus der US-A-4 066 494 ist die Verwendung von stickstoffhaltigen Kondensationsprodukten auf Basis von Polyalkylenpolyaminen als Entwässerungsbeschleuniger und Retentionsmittel in der Papierindustrie bekannt. Die stickstoffhaltigen Kondensationsprodukte werden durch Umsetzung von Polyalkylenpolyaminen, die 15 bis 500 Alkylenimin-Einheiten enthalten, mit α,ω-Chlorhydrinethern von Polyethylenoxyden, die 18 bis 90 Ethylenoxyd-Einheiten enthalten, bei 20 bis 100°C unter Bildung hochmolekularer, noch wasserlöslicher Harze hergestellt.

Aus der DE-C-2 916 356 ist ein Verfahren zur Herstellung von wasserlöslichen Polyetheraminen bekannt, bei dem man Di- oder Polyamine mit 2 bis 10 Stickstoffatomen mit Chlorhydrinethern aus 1 Mol eines zweiwertigen Alkohols mit 2 bis 5 Kohlenstoffatomen, deren Ethoxylierungsprodukten, die bis zu 18 Ethylenoxyd-Einheiten enthalten, Glycerin oder Polyglycerin, das bis zu 15 Glycerineinheiten enthält, und mindestens 2 bis 8 Mol Epichlorhydrin zunächst in polaren, mit Wasser mischbaren Lösemitteln in Abwesenheit von Wasser oder unter weitgehendem Ausschluß von Wasser bei Temperaturen von 110 bis 200°C kondensiert und dann eine Alkalimetall- oder Erdalkalimetallbase in einer solchen Menge zugibt, um mindestens 20 % des bei der Kondensation entstehenden Chlorwasserstoffs zu neutralisieren. Im Anschluß daran erfolgt noch eine Nachkondensation. Die Kondensationsprodukte werden als Flockungs-, Retentions- und Entwässerungsmittel bei der Herstellung von Papier verwendet.

Aus der WO-A-94/12560 sind weitere wasserlösliche, Aminogruppen enthaltende Kondensate bekannt, die dadurch erhältlich sind, daß man Polyalkylenpolyamine, vorzugsweise Polyethylenimin, durch Umsetzung mit beispielsweise Monocarbonsäuren zunächst teilweise amidiert und die dabei entstehenden Reaktionsprodukte anschließend mit mindestens bifunktionellen Vernetzern zu vernetzten Polyalkylenpolyaminen reagieren läßt, die in 20 gew.-%iger wäßriger Lösung bei 20°C eine Viskosität von mindestens 100 mPas haben. Diese Polymeren werden ebenfalls als Entwässerungs-, Flockungs- und Retentionsmittel sowie als Fixiermittel bei der Herstellung von Papier verwendet.

Aus der WO-A-94/14873 sind ebenfalls wasserlösliche, Aminogruppen enthaltende Polymere bekannt, die dadurch erhältlich sind, daß man Michaeladditionsprodukte von beispielsweise Polyalkylenpolyaminen, Polyamidoaminen oder mit Ethylenimin gepfropften Polyamidoaminen und monoethylenisch ungesättigten Carbonsäuren, Salzen, Estern, Amiden oder Nitrilen von monoethylenisch ungesättigten Carbonsäuren, mit mindestens bifunktionellen Vernetzern zu wasserlöslichen Kondensationsprodukten reagieren läßt, die in 20 gew.-%iger wäßriger Lösung bei 20°C eine Viskosität von mindestens 100 mPas haben. Die Polymeren werden als Entwässerungs-, Flockungs- und Retentionsmittel bei der Herstellung von Papier verwendet.

Aus Journal of Applied Polymer Science, Vol. 30, 4099 - 4111 (1985) ist bekannt, Polyamidoamin-Epichlorhydrin-Harze, die zur Antifilzausrüstung von Wolle (shrink-resist wool) oder auch als Naßfestmittel bei der Herstellung von Papier verwendet werden, durch Ultrafiltration in mehrere Fraktionen zu trennen. Prüft man die bei der Ultrafiltration erhaltenen beiden Hauptfraktionen auf ihre Wirksamkeit bei der Antifilzausrüstung von Wolle, so stellt man nur geringfügige Unterschiede fest. So sind beispielsweise die Eigenschaften von chlorierter Wolle, die jeweils mit einer der beiden bei der Ultrafiltration erhaltenen Fraktionen und mit einer nicht fraktionierten Probe des Polyamidoamin-Epichlorhydrin-Harzes behandelt wurden, sehr ähnlich. Daher wird gefolgert, daß eine Verfahrensänderung bei der Herstellung solcher Harze zur Erhöhung des Anteils einer Fraktion nicht mit einem praktischen Vorteil für die Antifilzausrüstung von Wolle verbunden ist.

Der Erfindung liegt die Aufgabe zugrunde, ein Verfahren zur Herstellung von wasserlöslichen, Aminogruppen enthaltenden Kondensaten und Additionsprodukten zur Verfügung zu stellen, wobei man Produkte erhält, die gegenüber vergleichbaren Produkten des Standes der Technik eine erhöhte Wirksamkeit bei der Anwendung aufweisen, z.B. die eine erhöhte Entwässerungs- und Retentionswirkung bei der Papierherstellung haben.

Die Aufgabe wird erfindungsgemäß gelöst mit einem Verfahren zur Herstellung von wasserlöslichen, Aminogruppen enthaltenden Kondensaten und Additionsprodukten mit erhöhter Entwässerungs- und Retentionswirkung bei der Herstellung von Papier, wenn man wäßrige Lösungen von Kondensaten oder Additionsprodukten aus der Gruppe der
- Reaktionsprodukte von Alkylendiaminen, Polyalkylenpolyaminen, mit Ethylenimin gepfropften Polyamidoaminen sowie deren Mischungen mit mindestens zwei funktionelle Gruppen aufweisenden Vernetzern,
- Reaktionsprodukte von Michaeladditionsprodukten aus Polyalkylenpolyaminen, Polyamidoaminen, mit Ethylenimin gepfropften Polyamidoaminen sowie deren Mischungen und monoethylenisch ungesättigten Carbonsäuren sowie deren Salzen, Estern, Amiden oder Nitrilen mit mindestens bifunktionellen Vernetzern,
- amidierten Polyethylenimine, die erhältlich sind durch Reaktion von Polyethyleniminen mit einbasischen Carbonsäuren oder ihren Estern, Anhydriden, Säurechloriden oder Säureamiden und gegebenenfalls Umsetzung der amidierten Polyethylenimine mit mindestens zwei funktionelle Gruppen aufweisenden Vernetzern,
- Polyethylenimine, quaternisierten Polyethylenimine, phosphonomethylierten Polyethylenimine, alkoxylierten Polyethylenimine und/oder durch Streckerreaktion carboxymethylierten Polyethylenimine und
- vernetzten alkoxylierten Polyethylenimine, vernetzten, quaternisierten Polyethylenimine, vernetzten, phosphonomethylierten Polyethylenimine und/oder vernetzten, durch Streckerreaktion carboxymethylierten Polyethylenimine
einer Ultrafiltration an Membranen unterwirft, wobei man 5 bis 95 Gew.-% der Kondensate oder Additionsprodukte als Permeat abtrennt und die wasserlöslichen, Aminogruppen enthaltenden Kondensate oder Additionsprodukte mit verbesserter Wirksamkeit gegebenenfalls aus dem Retentat isoliert.

Für die Ultrafiltration kann man sämtliche im Handel erhältlichen Membranen einsetzen, die eine Trenngrenze (auch als Ausschlußgrenze oder Cut-off-Grenze bezeichnet) für Polymere mit Molmassen von z.B. 1000 bis 10 Millionen, vorzugsweise von 1500 bis 500000 haben. Besonders bevorzugt verwendet man Membranen mit Trenngrenzen für Molmassen von 3000 bis 300 000. Die Trenngrenze der jeweils verwendeten Membranen ist der Molgewichtsverteilung der wasserlöslichen Aminogruppen enthaltenden Kondensate oder Additionsprodukte, die im folgenden Text der Einfachheit halber jeweils auch als wasserlösliche Polymere oder als Polymere bezeichnet werden, so anzupassen, daß die Abtrennung von 5 bis 95 Gew.-% an Polymer möglich ist. Bei der Ultrafiltration werden dann die niedermolekularen Anteile der Polymeren, deren Molmasse unterhalb der Trenngrenze liegt, als Permeat abgetrennt. Im Retentat verbleiben die höhermolekularen Anteile an Polymer. Die Ultrafiltration umfaßt gemäß der vorliegenden Erfindung auch die Mikrofiltration an Membranen, wobei man Membranen mit einem mittleren Porendurchmesser von 0,01 bis 10 µm, bevorzugt 0,1 bis 1 µm einsetzt. Durch die Abtrennung von niedermolekularen Anteilen erhält man Aminogruppen enthaltende wasserlösliche Kondensate oder Additionsprodukte mit engerer Molmassenverteilung und mit einer in nicht vorhersehbarer Weise gesteigerten Wirksamkeit z.B. beim Einsatz als Prozeßhilfsmittel bei der Herstellung von Papier.

Die Membranen können beispielsweise in Form von Rohren, Hohlfasern, Plattengeräten oder Spiral-Wickel-Modulen eingesetzt werden. Geeignete Materialien für die Membranen sind beispielsweise regenerierte Cellulose, Celluloseacetat, Cellulosetriacetat, Polyamid, Polyacrylnitril, Acrylnitrilcopolymerisate, Polysulfon, Copolymerisate des Vinylchlorids, Polyimid, Polyvinylidenfluorid, Polytetrafluorethylen, Polymethylmethacrylat, hydrolysierte Copolymerisate aus Ethylen und Vinylacetat, mit einem Hydrolysegrad der Vinylacetat-Gruppen von mindestens 50 %, Polycarbonat, Polyethylen, das nach dem Verfahren der Hochdruckpolymerisation von Ethylen hergestellt wird sowie HDPE (Polyethylen mit sehr hoher Dichte), Polypropylen, mineralische oder keramische Membranen oder insbesondere mechanisch stabile Membranen wie metallische Membranen, z.B. Membranen aus Edelstahl, die gegebenenfalls mit einer Sekundärmembran verbunden sind. Die Sekundärmembran kann aus Titanoxid oder Zirkonoxid oder aus einem organischen Material wie Polysulfon, bestehen. Vorzugsweise werden Membranen auf Basis von Polysulfon eingesetzt. Über Ultrafiltration und dafür geeignete Membranen wird beispielsweise zusammenfassend berichtet von Munir Cheryan in Ultrafiltration Handbook, Technomic Publishing Company, Inc., 1986.

Membranen, die für die Ultrafiltration geeignet sind, werden von zahlreichen Firmen angeboten, vgl. Katalog Internationales Treffen für chemische Technik und Biotechnologie ACHEMA 94, Frankfurt am Main.

Die Ultrafiltration der wäßrigen Lösungen von Aminogruppen enthaltenden Polymeren mit breiter Molmassenverteilung wird in üblicher Weise durchgeführt. Vorzugsweise arbeitet man bei Raumtemperatur und setzt wäßrige Lösungen von Ethylenimin-Einheiten enthaltenden Polymeren ein. Die Temperatur der wäßrigen Polymerlösungen kann z.B. in dem Bereich von 10 bis 80°C liegen. Die Ultrafiltration kann unter Atmosphärendruck oder auch unter erhöhtem Druck auf der Seite des Retentats durchgeführt werden, z.B. bei Drücken von 1,5 bis 50, vorzugsweise 2 bis 20 bar. Der pH-Wert der wäßrigen Lösungen von Aminogruppen enthaltenden Polymeren beträgt bei der Ultrafiltration z.B. 2 bis 14, vorzugsweise 4 bis 12 und insbesondere 8 bis 12.

Die Ultrafiltration der wasserlöslichen, Aminogruppen enthaltenden Polymeren, die eine breite Molmassenverteilung haben, wird in der Weise durchgeführt, daß man 5 bis 95, vorzugsweise 20 bis 90 Gew.-%, bezogen auf die eingesetzten Polymeren, an Polymeren als Permeat abtrennt und die Polymeren mit höherer Molmasse und überraschend verbesserter Qualität gegebenenfalls aus dem Retentat isoliert. Die Menge des als Permeat abgetrennten niedermolekularen Polymeren beträgt meistens 30 bis 70 Gew.-%.

Wie festgestellt wurde, weisen die bei dem erfindungsgemäßen Verfahren eingesetzten wasserlöslichen, Aminogruppen enthaltenden Kondensate und Additionsprodukte eine breite Molmassenverteilung M_{w}/Mₙ auf. So haben beispielsweise mit Ethylenimin gepfropfte, vernetzte Polyamidoamine eine Molmassenverteilung M_{w}/Mₙ von 400. Die Bestimmung der Molmassenverteilung erfolgt durch Gelpermeationschromatographie bezogen auf Pullulan-Standards mit Wasser als Elutionsmittel. Die erfindungsgemäß hergestellten Retentate haben beispielsweise Viskositäten von 120 bis 5000, vorzugsweise 200 bis 1500 mPas (gemessen in einem Brookfield-Viskosimeter an 10 gew.-%igen Polymerlösungen bei 20°C und pH 10) und eine Molmassenverteilung M_{w}/Mₙ von beispielsweise 2 bis 350, vorzugsweise 10 bis 300.

Nach dem erfindungsgemäßen Verfahren können sämtliche wasserlöslichen, Aminogruppen enthaltenden Polymeren, die üblicherweise eine breite Molmassenverteilung aufweisen, in enger verteilte Polymere mit hochmolekularen Anteilen und in Polymere mit niedermolekularen Anteilen getrennt werden. Aminogruppen enthaltende Polymerisate sind bekannt. Sie wurden im wesentlichen bereits oben als Stand der Technik beschrieben. Es handelt sich dabei z.B. um Reaktionsprodukte von Alkylendiaminen oder Polyalkylenpolyaminen mit mindestens zwei funktionelle Gruppen enthaltenden Vernetzern.

Zu dieser Gruppe gehören auch mit Ethylenimin gepfropfte Polyamidoamine, die in der zum Stand der Technik genannten US-A 4 144 123 beschrieben werden. Die Polyamidoamine werden beispielsweise durch Umsetzung von Dicarbonsäuren mit 4 bis 10 Kohlenstoffatomen mit Polyalkylenpolyaminen, die vorzugsweise 3 bis 10 basische Stickstoffatome im Molekül enthalten, hergestellt. Geeignete Dicarbonsäuren sind beispielsweise Bernsteinsäure, Maleinsäure, Adipinsäure, Glutarsäure, Korksäure, Sebazinsäure oder Terephthalsäure. Man kann auch Mischungen aus Adipinsäure und Glutarsäure oder Maleinsäure und Adipinsäure einsetzen. Bevorzugt verwendet man Adipinsäure zur Herstellung der Polyamidoamine.

Geeignete Polyalkylenpolyamine, die mit den Dicarbonsäuren kondensiert werden, sind beispielsweise Diethylentriamin, Triethylentetramin, Dipropylentriamin, Tripropylentetramin, Dihexamethylentriamin, Aminopropylethylendiamin und Bis-Aminopropylethylendiamin. Die Polyalkylenpolyamine können auch in Form von Mischungen bei der Herstellung der Polyamidoamine eingesetzt werden. Die Polyamidoamine werden vorzugsweise durch Kondensation von Dicarbonsäuren und Polyaminen in Substanz hergestellt. Die Kondensation kann jedoch gegebenenfalls auch in inerten Lösemitteln vorgenommen werden. Die Kondensation der Dicarbonsäuren mit den Polyalkylenpolyaminen wird üblicherweise in dem Temperaturbereich von beispielsweise 100 bis 220°C durchgeführt, wobei man das bei der Reaktion entstehende Wasser aus dem Reaktionsgemisch abdestilliert. Die Kondensation kann gegebenenfalls auch zusätzlich in Gegenwart von Lactonen oder Lactamen von Carbonsäuren mit 4 bis 8 Kohlenstoffatomen vorgenommen werden, z.B. in Gegenwart von Caprolactam. Pro Mol einer Dicarbonsäure verwendet man beispielsweise 0,8 bis 1,4 Mol eines Polyalkylenpolyamins. Die so erhältlichen Polyamidoamine weisen primäre und sekundäre NH-Gruppen, gegebenenfalls auch tertiäre Stickstoffatome auf und sind in Wasser löslich.

Die oben beschriebenen Polyamidoamine werden mit Ethylenimin gepfropft, indem man beispielsweise Ethylenimin in Gegenwart von Säuren (z.B. Schwefelsäure oder Phosphorsäure) oder in Gegenwart von Lewis-Säuren (z.B. Bortrifluoridetheraten) auf die Polyamidoamine einwirken läßt. Beispielsweise kann man pro basischer Stickstoffgruppierung im Polyamidoamin 1 bis 50, vorzugsweise 2 bis 25 Ethylenimin-Einheiten aufpfropfen, d.h. auf 100 Gewichtsteile eines Polyamidoamins setzt man beispielsweise etwa 10 bis 500 Gewichtsteile Ethylenimin ein. Diese Reaktionsprodukte können der Ultrafiltration unterworfen werden. Bevorzugt kommen jedoch Reaktionsprodukte in Betracht, die durch Pfropfen von Polyamidoaminen mit Ethylenimin und anschließende Reaktion der gepfropften Produkte mit mindestens zwei funktionelle Gruppen enthaltenden Vernetzern herstellbar sind. Produkte dieser Art werden beispielsweise gemäß der Lehre der oben genannten US-A-4 144 123 als Retentions-, Flockungs- und Entwässerungsmittel bei der Herstellung von Papier verwendet. Als Vernetzer kommen z.B. Bischlorhydrinether oder Bisglycidylether von Polyalkylenglykolen in Betracht, die 8 bis 100, vorzugsweise 15-50 Alkylenoxid-Einheiten enthalten. Zur Herstellung der wasserlöslichen Aminogruppen enthaltenden Kondensate setzt man z.B. ein Gew.-Teil eines mit Ethylenimin gepfropften Polyamidoamins mit 0,3 bis 2 Gew.-Teilen der Bisglycidylether oder der anderen mindestens zwei funktionelle Gruppen aufweisenden Vernetzer um. Besonders bevorzugt eingesetzte Kondensate sind Reaktionsprodukte, die durch Vernetzung von mit Ethylenimin gepfropften Polyamidoaminen mit Bisglycidylethern von Polyalkylenglykolen, die 8 bis 100 Ethylenoxid- und/ oder Propylenoxid-Einheiten enthalten, erhältlich sind.

Außer den in der US-A 4 144 123 beschriebenen Bischlorhydrin- oder Glycidylethern von Polyalkylenglykolen als mindestens zwei funktionelle Gruppen enthaltenden Vernetzer eignen sich α,ω-Dichlorpolyalkylenglykole, die beispielsweise als Vernetzer aus der EP-B-0 025 515 bekannt sind. Sie sind dadurch erhältlich, daß man zwei- bis vierwertige Alkohole, vorzugsweise alkoxylierte zweibis vierwertige Alkohole entweder mit Thionylchlorid unter HCl-Abspaltung und nachfolgender katalytischer Zersetzung der chlorsulfonierten Verbindungen unter Schwefeldioxidabspaltung umsetzt oder sie mit Phosgen unter HCl-Abspaltung in die entsprechenden Bischlorkohlensäureester überführt und daraus anschließend durch katalytische Zersetzung unter Kohlendioxidabspaltung α,ω-Dichlorether erhält. Als Alkoholkomponente werden vorzugsweise ethoxylierte und/oder propoxylierte Glykole eingesetzt, die mit 1 bis 100, insbesondere 4 bis 40 Mol Ethylenoxid pro Mol Glykol zur Reaktion gebracht werden.

Andere geeignete Vernetzer sind α,ω- oder vicinale Dichloralkane, beispielsweise 1,2-Dichlorethan, 1,2-Dichlorpropan, 1,3-Dichlorpropan, 1,4-Dichlorbutan und 1,6-Dichlorhexan. Beispiele für weitere Vernetzer sind die Umsetzungsprodukte von mindestens dreiwertigen Alkoholen mit Epichlorhydrin zu Reaktionsprodukten, die mindestens zwei Chlorhydrin-Einheiten aufweisen, z.B. verwendet man als mehrwertige Alkohole Glycerin, ethoxilierte oder propoxilierte Glycerine, Polyglycerine mit 2 bis 15 Glycerin-Einheiten im Molekül sowie gegebenenfalls ethoxilierte und/oder propoxilierte Polyglycerine. Vernetzer dieser Art sind beispielsweise aus der DE-A-2 916 356 bekannt. Außerdem eignen sich Vernetzer, die blockierte Isocyanat-Gruppen enthalten, z.B. Trimethylhexamethylendiisocyanat blockiert mit 2,2,3,6-Tetramethylpiperidinon-4. Solche Vernetzer sind bekannt, vgl. beispielsweise DE-A-4 028 285, sowie Aziridin-Einheiten enthaltende Vernetzer auf Basis von Polyethern oder substituierten Kohlenwasserstoffen, z.B. 1,6-Bis-N-aziridinohexan, vgl. US-A-3 977 923. Es ist selbstverständlich auch möglich, Mischungen aus zwei oder mehreren Vernetzern zur Molekulargewichtserhöhung zu verwenden.

Bei der Papierherstellung werden insbesondere solche Prozeßhilfsmittel bevorzugt, die frei von organisch gebundenem Halogen sind. Daher werden zur Herstellung von Aminogruppen enthaltenden Kondensaten oder von Aminogruppen enthaltenden Additionsprodukten, die jeweils in Wasser löslich sind, halogenfreie Vernetzer verwendet. Die halogenfreien Vernetzer sind mindestens bifunktionell und sind vorzugsweise ausgewählt aus der Gruppe bestehend aus:
(1) Ethylencarbonat, Propylencarbonat und/oder Harnstoff,
(2) monoethylenisch ungesättigten Carbonsäuren und deren Estern, Amiden und Anhydriden, mindestens zweibasischen gesättigten Carbonsäuren oder Polycarbonsäuren sowie den jeweils davon abgeleiteten Estern, Amiden und Anhydriden,
(3) Umsetzungsprodukten von Polyetherdiaminen, Alkylendiaminen, Polyalkylenpolyaminen, Alkylenglykolen, Polyalkylenglykolen oder deren Gemischen mit monoethylenisch ungesättigten Carbonsäuren, Estern, Amiden oder Anhydriden monoethylenisch ungesättigter Carbonsäuren, wobei die Umsetzungsprodukte mindestens zwei ethylenisch ungesättigte Doppelbindungen, Carbonsäureamid-, Carboxyl- oder Estergruppen als funktionelle Gruppen aufweisen,
(4) mindestens zwei Aziridinogruppen enthaltenden Umsetzungsprodukten von Dicarbonsäureestern mit Ethylenimin
   sowie Mischungen der genannten Vernetzer.

Geeignete Vernetzer der Gruppe (1) sind Ethylencarbonat, Propylencarbonat und Harnstoff. Aus dieser Gruppe von Monomeren wird vorzugsweise Propylencarbonat verwendet. Die Vernetzer dieser Gruppe reagieren zu Aminogruppen enthaltenden Harnstoffverbindungen.

Geeignete halogenfreie Vernetzer der Gruppe (2) sind z.B. monoethylenisch ungesättigte Monocarbonsäuren wie Acrylsäure, Methacrylsäure und Crotonsäure sowie die davon abgeleiteten Amide, Ester und Anhydride. Die Ester können sich von Alkoholen mit 1 bis 22, vorzugsweise 1 bis 18 C-Atomen ableiten. Die Amide sind vorzugsweise unsubstituiert, können jedoch einen C₁- bis C₂₂-Alkylrest als Substituent tragen.

Weitere halogenfreie Vernetzer der Gruppe (2) sind mindestens zweibasische gesättigte Carbonsäuren wie Dicarbonsäuren sowie die davon abgeleiteten Salze, Diester und Diamide. Diese Verbindungen können beispielsweise mit Hilfe der Formel 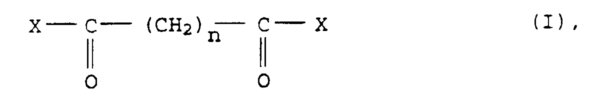 in der
X = OH, OR, 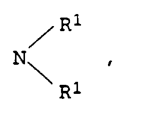
R = C₁- bis C₂₂-Alkyl,
R¹ = H, C₁- bis C₂₂-Alkyl und
n = 0 bis 22
charakterisiert werden. Außer den Dicarbonsäuren der Formel I eignen sich beispielsweise monoethylenisch ungesättigte Dicarbonsäuren wie Maleinsäure oder Itaconsäure. Die Ester der in Betracht kommenden Dicarbonsäuren leiten sich vorzugsweise von Alkoholen mit 1 bis 4 Kohlenstoffatomen ab. Geeignete Dicarbonsäureester sind beispielsweise Oxalsäuredimethylester, Oxalsäurediethylester, Oxalsäurediisopropylester, Bernsteinsäuredimethylester, Bernsteinsäurediethylester, Bernsteinsäurediisopropylester, Bernsteinsäuredi-n-propylester, Bernsteinsäurediisobutylester, Adipinsäuredimethylester, Adipinsäurediethylester und Adipinsäurediisopropylester. Geeignete Ester von ethylenisch ungesättigten Dicarbonsäuren sind beispielsweise Maleinsäuredimethylester, Maleinsäurediethylester, Maleinsäurediisopropylester, Itaconsäuredimethylester und Itaconsäurediisopropylester. Außerdem kommen substituierte Dicarbonsäuren und ihre Ester wie Weinsäure (D-, L-Form und als Racemat) sowie Weinsäureester, wie Weinsäuredimethylester und Weinsäurediethylester in Betracht.

Geeignete Dicarbonsäureanhydride sind beispielsweise Maleinsäureanhydrid, Itaconsäureanhydrid und Bernsteinsäureanhydrid. Die Vernetzung von Aminogruppen enthaltenden Verbindungen der Komponente (a) mit den vorstehend genannten halogenfreien Vernetzern erfolgt unter Bildung von Amidgruppen bzw. bei Amiden wie Adipinsäurediamid durch Umamidierung. Maleinsäureester, monoethylenisch ungesättigte Dicarbonsäuren sowie deren Anhydride können sowohl durch Bildung von Carbonsäureamidgruppen als auch durch Addition von NH-Gruppen der zu vernetzenden Komponente (z,B, von Polyamidoaminen) nach Art einer Michael-Addition eine Vernetzung bewirken.

Zu mindestens zweibasischen gesättigten Carbonsäuren gehören beispielsweise Tri- und Tetracarbonsäuren wie Citronensäure, Propantricarbonsäure, Ethylendiamintetraessigsäure und Butantetracarbonsäure. Als Vernetzer der Gruppe (2) kommen außerdem die von den vorstehend genannten Carbonsäuren abgeleiteten Salze, Ester, Amide und Anhydride in Betracht.

Geeignete Vernetzer der Gruppe (2) sind außerdem Polycarbonsäuren, die durch Polymerisieren von monoethylenisch ungesättigten Carbonsäuren oder Anhydriden erhältlich sind. Als monoethylenisch ungesättigte Carbonsäuren kommen z.B. Acrylsäure, Methacrylsäure, Fumarsäure, Maleinsäure und/oder Itaconsäure in Betracht. So eignen sich als Vernetzer z.B. Polyacrylsäuren, Copolymerisate aus Acrylsäure und Methacrylsäure oder Copolymerisate aus Acrylsäure und Maleinsäure.

Weitere geeignete Vernetzer (2) werden z.B. durch Polymerisieren von Anhydriden wie Maleinsäureanhydrid in einem inerten Lösemittel wie Toluol, Xylol, Ethylbenzol, Isopropylbenzol oder Lösemittelgemischen in Gegenwart von Radikalen bildenden Initiatoren hergestellt. Als Initiatoren verwendet man vorzugsweise Peroxyester wie tert.-Butyl-per-2-ethylhexanoat. Außer den Homopolymerisaten kommen Copolymerisate von Maleinsäureanhydrid in Betracht, z.B. Copolymerisate aus Acrylsäure und Maleinsäureanhydrid oder Copolymerisate aus Maleinsäureanhydrid und einem C₂-bis C₃₀-Olefin.

Bevorzugt sind beispielsweise Copolymerisate aus Maleinsäureanhydrid und Isobuten oder Copolymerisate aus Maleinsäureanhydrid und Diisobuten. Die Anhydridgruppen enthaltenden Copolymerisate können gegebenenfalls durch Umsetzung mit C₁- bis C₂₀-Alkoholen oder Ammoniak oder Aminen modifiziert sein und in dieser Form als Vernetzer eingesetzt werden.

Die Molmasse M_{w} der Homo- und Copolymeren beträgt z.B. bis zu 10000, vorzugsweise 500 bis 5000. Polymerisate der oben genannten Art werden z.B. beschrieben in EP-A-0 276 464, US-A-3 810 834, GB-A-1 411 063 und US-A-4 818 795. Die mindestens zweibasischen gesättigten Carbonsäuren und die Polycarbonsäuren können auch in Form der Alkali- oder Ammoniumsalze als Vernetzer eingesetzt werden. Bevorzugt verwendet man dabei die Natriumsalze. Die Polycarbonsäuren können partiell, z.B. zu 10 bis 50 mol-% oder auch vollständig neutralisiert sein.

Bevorzugt eingesetzte Verbindungen der Gruppe (2) sind Weinsäuredimethylester, Weinsäurediethylester, Adipinsäuredimethylester, Adipinsäurediethylester, Maleinsäuredimethylester, Maleinsäurediethylester, Maleinsäureanhydrid, Maleinsäure, Acrylsäure, Acrylsäuremethylester, Acrylsäureethylester, Acrylamid und Methacrylamid.

Halogenfreie Vernetzer der Gruppe (3) sind beispielsweise Umsetzungsprodukte von Polyetherdiaminen, Alkylendiaminen, Polyalkylenpolyaminen, Alkylenglykolen, Polyalkylenglykolen oder deren Gemischen mit
- monoethylenisch ungesättigten Carbonsäuren,
- Estern monoethylenisch ungesättigter Carbonsäuren,
- Amiden monoethylenisch ungesättigter Carbonsäuren oder
- Anhydriden monoethylenisch ungesättigter Carbonsäuren.

Die Polyetherdiamine werden beispielsweise durch Umsetzung von Polyalkylenglykolen mit Ammoniak hergestellt. Die Polyalkylenglykole können 2 bis 50, vorzugsweise 2 bis 40 Alkylenoxideinheiten enthalten. Hierbei kann es sich beispielsweise um Polyethylenglykole, Polypropylenglykole, Polybutylenglykole oder auch um Blockcopolymerisate aus Ethylenglykol und Propylenglykol, Blockcopolymerisate aus Ethylenglykol und Butylenglykol oder um Blockcopolymerisate aus Ethylenglykol, Propylenglykol und Butylenglykol handeln. Außer den Blockcopolymerisaten eignen sich zur Herstellung der Polyetherdiamine statistisch aufgebaute Copolymerisate aus Ethylenoxid und Propylenoxid und gegebenenfalls Butylenoxid. Polyetherdiamine leiten sich außerdem von Polytetrahydrofuranen ab, die 2 bis 75 Tetrahydrofuraneinheiten aufweisen. Die Polytetrahydrofurane werden ebenfalls durch Umsetzung mit Ammoniak in die entsprechenden α,ω-Polyetherdiamine überführt. Vorzugsweise verwendet man zur Herstellung der Polyetherdiamine Polyethylenglykole oder Blockcopolymerisate aus Ethylenglykol und Propylenglykol.

Als Alkylendiamine kommen beispielsweise Ethylendiamin, Propylendiamin, 1,4-Diaminobutan und 1,6-Diaminohexan in Betracht. Geeignete Polyalkylenpolyamine sind beispielsweise Diethylentriamin, Triethylentetramin, Dipropylentriamin, Tripropylentetramin, Dihexamethylentriamin, Aminopropylethylendiamin, Bis-Aminopropylethylendiamin und Polyethylenimine mit Molmassen bis zu 5000. Die vorstehend beschriebenen Amine werden mit monoethylenisch ungesättigten Carbonsäuren, Estern, Amiden oder Anhydriden monoethylenisch ungesättigter Carbonsäuren so umgesetzt, daß die entstehenden Produkte mindestens 2 ethylenisch ungesättigte Doppelbindungen, Carbonsäureamid-, Carboxyl- oder Estergruppen als funktionelle Gruppen aufweisen. So erhält man beispielsweise bei der Umsetzung der in Betracht kommenden Amine oder Glykole mit Maleinsäureanhydrid Verbindungen, die beispielsweise mit Hilfe der Formel II charakterisiert werden können: 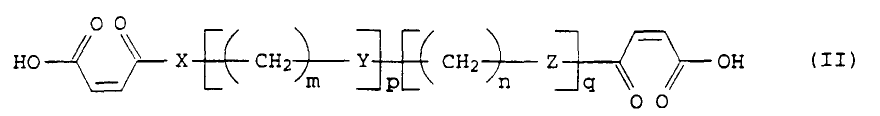 in der
X, Y, Z = O, NH
   und Y zusätzlich noch CH₂
m, n = 0 - 4
p, q = 0 - 45000
bedeuten.

Die Verbindungen der Formel (II) sind beispielsweise dadurch erhältlich, daß man Alkylenglykole, Polyethylenglykole, Polyethylenimine, Polypropylenimine, Polytetrahydrofurane, α,ω-Diole oder α,ω-Diamine mit Maleinsäureanhydrid oder den oben angegebenen anderen monoethylenisch ungesättigten Carbonsäuren bzw. Carbonsäurederivaten umsetzt. Die für die Herstellung der Vernetzer II in Betracht kommenden Polyethylenglykole haben vorzugsweise Molmassen von 62 bis 10000, die Molmassen der Polyethylenimine betragen vorzugsweise 129 bis 50000, die der Polypropylenimine 171 bis 50000. Als Alkylenglykole eignen sich z.B. Ethylenglykol, 1,2-Propylenglykol, 1,4-Butandiol und 1,6-Hexandiol.

Vorzugsweise eingesetzte α,ω-Diamine sind Ethylendiamin und von Polyethylenglykolen oder von Polytetrahydrofuranen mit Molmassen M_{w} von jeweils ca. 400 bis 5000 abgeleitete α,ω-Diamine,

Besonders bevorzugt in Betracht kommende Vernetzer der Formel II sind Umsetzungsprodukte von Maleinsäureanhydrid mit α,ω-Polyetherdiaminen einer Molmasse von 400 bis 5000, die Umsetzungsprodukte von Polyethyleniminen einer Molmasse von 129 bis 50000 mit Maleinsäureanhydrid sowie die Umsetzungsprodukte von Ethylendiamin oder Triethylentetramin mit Maleinsäureanhydrid im Molverhältnis von 1: mindestens 2. Bei der Umsetzung von Polyalkylenglykolen bzw. Diolen mit monoethylenisch ungesättigten Carbonsäuren, ihren Estern, Amiden oder Anhydriden entstehen unter Erhalt der Doppelbindung der monoethylenisch ungesättigten Carbonsäuren bzw. ihrer Derivate Vernetzer, bei denen die monoethylenisch ungesättigten Carbonsäuren bzw. ihre Derivate über eine Amidgruppe mit den Polyetherdiaminen, Alkylendiaminen oder Polyalkylenpolyaminen und über eine Estergruppe mit den Alkylenglykolen bzw. Polyalkylenglykolen verknüpft sind. Diese Umsetzungsprodukte enthalten mindestens zwei ethylenisch ungesättigte Doppelbindungen. Dieser Vernetzertyp reagiert mit den Aminogruppen der zu vernetzenden Verbindungen nach Art einer Michael-Addition an die endständigen Doppelbindungen dieser Vernetzer und gegebenenfalls zusätzlich unter Bildung von Amidgruppen.

Polyetherdiamine, Alkylendiamine und Polyalkylenpolyamine können mit Maleinsäureanhydrid oder den ethylenisch ungesättigten Carbonsäuren bzw. deren Derivaten auch unter Anlagerung an die Doppelbindung nach Art einer Michael-Addition reagieren. Hierbei erhält man Vernetzer der Formel III 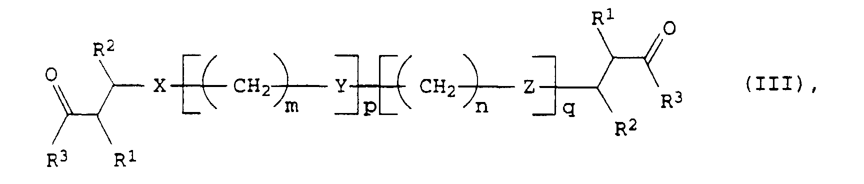 in der
X, Y, Z = O, NH
   und Y zusätzlich noch CH₂
R¹ = H, CH₃
R² = H, COOMe, COOR, CONH₂
R³ = OR, NH₂, OH, OMe
R = C₁- bis C₂₂-Alkyl
Me = H, Na, K, Mg, Ca

m, n = 0 - 4
p, q = 0 - 45000
bedeuten.

Die Vernetzer der Formel (III) bewirken über ihre endständigen Carboxyl- oder Estergruppen unter Ausbildung einer Amidfunktion eine Vernetzung mit den Aminogruppen enthaltenden Verbindungen. Zu dieser Klasse von Vernetzersystemen gehören auch die Umsetzungsprodukte von monoethylenisch ungesättigten Carbonsäureestern mit Alkylendiaminen und Polyalkylenpolyaminen, z.B. eignen sich die Additionsprodukte von Ethylendiamin, Diethylentriamin, Triethylentetramin, Tetraethylenpentamin sowie von Polyethyleniminen mit Molmassen von beispielsweise 129 bis 50000 an Acrylsäure- oder Methacrylsäureester, wobei man auf 1 Mol der Aminkomponente mindestens 2 Mol der Acrylsäure- oder Methacrylsäureester einsetzt. Als Ester monoethylenisch ungesättigter Carbonsäuren setzt man bevorzugt die C₁- bis C₆-Alkylester der Acrylsäure oder Methacrylsäure ein. Besonders bevorzugt zur Herstellung der Vernetzer werden Acrylsäuremethylester und Acrylsäureethylester. Die Vernetzer, die durch Michael-Addition von Polyalkylenpolyaminen und ethylenisch ungesättigten Carbonsäuren, Estern, Amiden oder Anhydriden hergestellt werden, können mehr als zwei funktionelle Gruppen aufweisen. Die Anzahl dieser Gruppen hängt davon ab, in welchem Mol-Verhältnis die Reaktionsteilnehmer bei der Michael-Addition eingesetzt werden. So kann man z.B. an ein Mol eines 10 Stickstoffatome enthaltenden Polyalkylenpolyamins 2 bis 10, vorzugsweise 2 bis 8 Mol an ethylenisch ungesättigten Carbonsäuren bzw. ihren Derivaten nach Art einer Michael-Addition anlagern. An jeweils 1 Mol Polyalkylendiamine und Alkylendiamine können mindestens 2 bis höchstens 4 Mol der ethylenisch ungesättigten Carbonsäuren bzw. ihrer Derivate nach Art einer Michael-Addition angelagert werden.

Bei der Umsetzung von Diethylentriamin und einer Verbindung der Formel 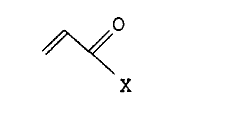 in der X = OH, NH₂ oder OR¹ und R¹= C₁- bis C₂₂-Alkyl bedeutet, entsteht nach Art einer Michaeladdition beipielsweise ein Vernetzer der Struktur 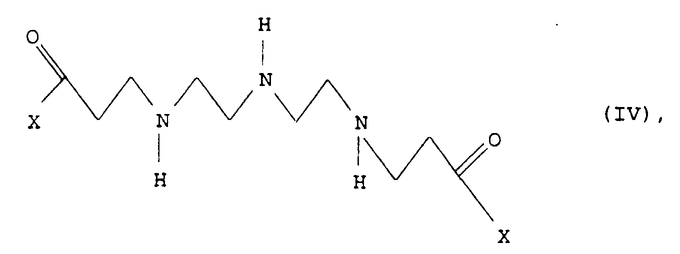 in der
X = NH₂, OH oder OR¹ und
R¹ für C₁- bis C₂₂-Alkyl steht.

Die sekundären NH-Gruppen in den Verbindungen der Formel IV können gegebenenfalls mit Acrylsäure, Acrylamid oder Acrylestern nach Art einer Michael-Addition reagieren.

Als Vernetzer der Gruppe (3) verwendet man vorzugsweise die Verbindungen der Formel II, die mindestens 2 Carboxylgruppen enthalten und durch Umsetzung von Polyetherdiaminen, Ethylendiamin oder Polyalkylenpolyaminen mit Maleinsäureanhydrid erhältlich sind oder mindestens 2 Estergruppen enthaltende Michael-Additionsprodukte aus Polyetherdiaminen, Polyalkylenpolyaminen oder Ethylendiamin und Estern der Acrylsäure oder Methacrylsäure mit jeweils einwertigen 1 bis 4 C-Atome enthaltenden Alkoholen.

Als halogenfreie Vernetzer der Gruppe (4) kommen Reaktionsprodukte in Betracht, die durch Umsetzung von Dicarbonsäureestern, die mit einwertigen Alkoholen mit 1 bis 5 Kohlenstoffatomen vollständig verestert sind, mit Ethylenimin hergestellt werden. Geeignete Dicarbonsäureester sind beispielsweise Oxalsäuredimethylester, Oxalsäurediethylester, Bernsteinsäuredimethylester, Bernsteinsäurediethylester, Adipinsäuredimethylester, Adipinsäurediethylester und Glutarsäuredimethylester. So erhält man beispielsweise bei der Umsetzung von Diethyloxalat mit Ethylenimin Bis-[β-(1-Aziridino)ethyl]oxalsäureamid. Die Dicarbonsäureester werden mit Ethylenimin beispielsweise im Molverhältnis von 1 zu mindestens 4 umgesetzt. Reaktive Gruppe dieser Vernetzer sind die endständigen Aziridingruppen. Diese Vernetzer können beispielsweise mit Hilfe der Formel V charakterisiert werden: 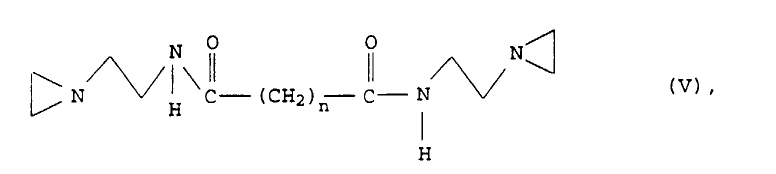 worin n = 0 bis 22 bedeutet.

Die oben beschriebenen Vernetzer können entweder allein oder in Mischung bei der Reaktion mit wasserlöslichen, Aminogruppen enthaltenden Polymeren, Diaminen oder Polyalkylenpolyaminen eingesetzt werden. Die Vernetzungsreaktion wird dabei in allen Fällen höchstens soweit geführt, daß die entstehenden Produkte noch wasserlöslich sind, z.B. sollen sich mindestens 10 g des vernetzten Polymeren in 1 l Wasser bei einer Temperatur von 20°C lösen. Die Vernetzungsreaktion erfolgt in bekannter Weise durch Erhitzen der Reaktionskomponenten bei Temperaturen von 50 bis 220, vorzugsweise bei Temperaturen von 60 bis 100°C. Sofern die Vernetzungsreaktion bei Temperaturen oberhalb von 100°C in wäßrigem Medium durchgeführt wird, benötigt man dafür druckdicht ausgelegte Apparaturen, z.B. einen mit einem Rührer ausgestatteten Autoklaven.

Als wasserlösliche, Aminogruppen enthaltende Polymere kommen außerdem Reaktionsprodukte in Betracht, die erhältlich sind durch Umsetzung von Michaeladditionsprodukten aus Polyalkylenpolyaminen, Polyamidoaminen, mit Ethylenimin gepfropften Polyamidoaminen sowie Mischungen der genannten Verbindungen und monoethylenisch ungesättigten Carbonsäuren, Salzen, Estern, Amiden oder Nitrilen mit mindestens bifunktionellen Vernetzern. Solche Reaktionsprodukte sind beispielsweise aus der WO-A-94/184743 bekannt. Zu ihrer Herstellung kommen außer den halogenhaltigen Vernetzern besonders die oben beschriebenen Klassen von halogenfreien Vernetzern in Betracht.

Eine weitere Klasse von Aminogruppen, vorzugsweise Ethylenimin-Einheiten enthaltenden Polymeren ist aus der WO-A-94/12560 bekannt. Es handelt sich hierbei um wasserlösliche, vernetzte, teilweise amidierte Polyethylenimine, die erhältlich sind durch
- Reaktion von Polyethyleniminen mit einbasischen Carbonsäuren oder ihren Estern, Anhydriden, Säurechloriden oder Säureamiden unter Amidbildung und
- Umsetzung der amidierten Polyethylenimine mit mindestens zwei funktionelle Gruppen enthaltenden Vernetzern.

Die einbasischen Carbonsäuren haben beispielsweise 1 bis 28, vorzugsweise 8 bis 18 C-Atome und können gegebenenfalls eine oder mehrere ethylenische Doppelbindungen enthalten, z.B. Ölsäure oder Linolensäure,

Die Molmassen der in Betracht kommenden Polyethylenimine können bis zu 2 Mio. betragen und liegen vorzugsweise in dem Bereich von 1000 bis 50000. Die Polyethylenimine werden partiell mit einbasischen Carbonsäuren amidiert, so daß beispielsweise 0,1 bis 90, vorzugsweise 1 bis 50 % der amidierbaren Stickstoffatome in den Polyethyleniminen als Amidgruppe vorliegt. Geeignete, mindestens zwei funktionelle Doppelbindungen enthaltende Vernetzer sind oben genannt. Vorzugsweise werden halogenfreie Vernetzer eingesetzt.

Bei der Reaktion von Aminogruppen enthaltenden Verbindungen mit Vernetzern setzt man beispielsweise auf 1 Gewichtsteil einer Aminogruppen enthaltenden Verbindung 0,001 bis 10, vorzugsweise 0,01 bis 3 Gewichtsteile mindestens eines Vernetzers ein.

Andere Aminogruppen enthaltende Additionsprodukte, die bei der Ultrafiltration eingesetzt werden sind Polyethylenimine sowie quaternisierte Polyethylenimine. Es kommen hierfür z.B. sowohl Homopolymerisate von Ethylenimin als auch Polymere in Betracht, die beispielsweise Ethylenimin aufgepfropft enthalten. Die Homopolymerisate werden beispielsweise durch Polymerisieren von Ethylenimin in wäßriger Lösung in Gegenwart von Säuren, Lewis-Säuren oder Alkylierungsmitteln wie Methylchlorid, Ethylchlorid, Propylchlorid, Ethylenchlorid, Chloroform oder Tetrachlorethylen hergestellt. Die so erhältlichen Polyethylenimine haben eine breite Molmassenverteilung und Molmassen von beispielsweise 129 bis 2.10⁶, vorzugsweise 430 bis 1.10⁶.

Die Polyethylenimine und die quaternisierten Polyethylenimine können gegebenenfalls mit einem mindestens zwei funktionelle Gruppen enthaltenden Vernetzer umgesetzt sein. Die Quaternisierung der Polyethylenimine kann beispielsweise mit Alkylhalogeniden wie Methylchlorid, Ethylchlorid, Hexylchlorid, Benzylchlorid oder Laurylchlorid sowie mit beispielsweise Dimethylsulfat vorgenommen werden. Weitere geeignete Aminogruppen enthaltende Polymere, deren Qualität durch Ultrafiltration verbessert werden kann, sind durch Strecker-Reaktion modifizierte Polyethylenimine, z.B. die Umsetzungsprodukte von Polyethyleniminen mit Formaldehyd und Natriumcyanid unter Hydrolyse der dabei entstehenden Nitrile zu den entsprechenden Carbonsäuren. Diese Produkte können gegebenenfalls mit einem mindestens zwei funktionelle Gruppen enthaltenden Vernetzer umgesetzt sein.

Außerdem eignen sich phosphonomethylierte Polyethylenimine und alkoxylierte Polyethylenimine, die beispielsweise durch Umsetzung von Polyethylenimin mit Ethylenoxid und/oder Propylenoxid erhältlich sind. Die phosphonomethylierten und die alkoxylierten Polyethylenimine können gegebenenfalls mit einem mindestens zwei funktionelle Gruppen enthaltenden Vernetzer umgesetzt sein. Die alkoxylierten Polyethylenimine enthalten pro NH-Gruppe 1 bis 100, vorzugsweise 2 bis 20 Alkylenoxid-Einheiten. Die Molmasse der Polyethylenimine kann bis zu 2 Millionen betragen. Vorzugsweise verwendet man für die Alkoxylierung Polyethylenimine mit Molmassen von 1000 bis 50000. Weitere geeignete wasserlösliche Aminogruppen enthaltende Polymere sind Umsetzungsprodukte von Polyethyleniminen mit Diketenen, z.B. von Polyethyleniminen einer Molmasse von 1000 bis 50000 mit Distearyldiketen. Auch solche Produkte können gegebenenfalls mit einem mindestens zwei funktionelle Gruppen enthaltenden Vernetzer umgesetzt sein. Weitere geeignete wasserlösliche Kondensationsprodukte sind vernetzte, durch Streckerreaktion carboxymethylierte Polyethylenimine, die durch Umsetzung von carboxymethylierten Polyethyleniminen mit mindestens einem der obengenannten Vernetzer erhältlich sind.

Die oben beschriebenen wasserlöslichen Aminogruppen enthaltenden Polymeren mit breiter Molmassenverteilung werden erfindungsgemäß der Ultrafiltration unterworfen. Die hierbei als Permeat abgetrennten niedermolekularen Anteile an Kondensaten oder Additionsprodukten können in den Herstellungsprozeß, der bei der Ultrafiltration als Ausgangsprodukte eingesetzten wasserlöslichen Polymeren zurückgeführt werden, d.h. sie werden zur Synthese von breit verteilten, wasserlöslichen, Aminogruppen enthaltenden Polymeren eingesetzt, die dann erneut der Ultrafiltration an Membranen unterworfen werden, wobei man 5 bis 95 Gew.-%, bezogen auf die eingesetzten Polymeren, an niedermolekularen Polymeren als Permeat abtrennt. Die niedermolekularen Anteile können somit aus den wasserlöslichen Aminogruppen enthaltenden Polymeren mit breiter Molmassenverteilung abgetrennt und nach einer Erhöhung der Molmasse in den Prozeß der Ultrafiltration zurückgeführt werden. Sofern die Konzentration der abgetrennten niedermolekularen Anteile an Polymeren im Permeat zu gering ist, wird das Permeat durch Abdestillieren von Wasser aufkonzentriert, z.B. stellt man eine Konzentration von 5 bis 50, vorzugsweise 10 bis 30 Gew.-% an Polymer ein. Die in dem abgetrennten Permeat enthaltenen niedermolekularen Anteile an Polymeren können beispielsweise mit Ethylenimin gepfropft werden. Die Umsetzung wird im wäßrigen Medium beispielsweise bei Temperaturen von 50 bis 200°C unter Einwirkung von Säuren wie Schwefelsäure oder Phosphorsäure oder von Lewis-Säuren durchgeführt.

Die bei der Ultrafiltration mit dem Permeat abgetrennten niedermolekularen Anteile an Aminogruppen enthaltenden Polymeren können auch mit mindestens zwei funktionelle Gruppen aufweisenden Vernetzern zu höhermolekularen Polymeren mit breiter Molmassenverteilung umgesetzt werden, die dann erneut der Ultrafiltration unterworfen werden. Geeignete Vernetzer sind bereits oben angegeben worden.

Die bei der Ultrafiltration abgetrennten niedermolekularen Anteile an Polymeren können auch dahingehend zu Produkten mit breiter Molmassenverteilung aufgearbeitet werden, daß man sie zunächst mit Ethylenimin umsetzt und die dabei erhältlichen Reaktionsprodukte mit mindestens zwei funktionelle Gruppen aufweisenden Vernetzern reagieren läßt. Diese so erhältlichen Polymeren mit breiter Molmassenverteilung werden in die Ultrafiltration zurückgeführt, um daraus die hochmolekularen Anteile mit engerer Molmassenverteilung und einer Viskosität von mindestens 120 mPas (gemessen in 10 gew.-%iger wäßriger Lösung bei 20°C und pH 10) zu gewinnen. Die als Retentat anfallenden höher molekularen bzw. hochmolekularen Anteile an Ethylenimin-Einheiten enthaltenden Polymeren können gegebenenfalls aus den wäßrigen Lösungen isoliert werden, indem man z.B. das Wasser abdampft. Der überwiegende Teil der Ethylenimin-Einheiten enthaltenden Polymeren mit engerer Molmassenverteilung wird jedoch in Form wäßriger Lösungen angewendet. Die Konzentration der wäßrigen Lösungen beträgt beispielsweise im Fall der mit Ethylenimin gepfropften und vernetzten Polyamidoamine 5 bis 25, vorzugsweise 7 bis 20 Gew.-%.

Besonders bevorzugt werden als wasserlösliche Aminogruppen enthaltende Kondensate und/oder Additionsprodukte solche Reaktionsprodukte eingesetzt, die durch Pfropfen von Polyamidoaminen mit Ethylenimin und anschließende Reaktion mit mindestens zwei funktionelle Gruppen enthaltenden Vernetzern erhältlich sind. Als Vernetzer kommen hierfür vorzugsweise halogenfreie bifunktionelle Verbindungen in Betracht, die ausgewählt sind aus der Gruppe bestehend aus
(1) Ethylencarbonat, Propylencarbonat und/oder Harnstoff,
(2) monoethylenisch ungesättigten Carbonsäuren und deren Estern, Amiden und Anhydriden, mindestens zweibasischen gesättigten Carbonsäuren oder Polycarbonsäuren sowie den jeweils davon abgeleiteten Estern, Amiden und Anhydriden.
(3) Umsetzungsprodukten von Polyetherdiaminen, Alkylendiaminen, Polyalkylenpolyaminen, Alkylenglykolen, Polyalkylenglykolen oder deren Gemischen mit monoethylenisch ungesättigten Carbonsäuren, Estern, Amiden oder Anhydriden monoethylenisch ungesättigter Coarbonsäuren, wobei die Umsetzungsprodukte mindestens zwei ethylenisch ungesättigte Doppelbindungen, Carbonsäureamid-, Carboxyl- oder Estergruppen als funktionelle Gruppen aufweisen,
(4) mindestens zwei Aziridinogruppen enthaltenden Umsetzungsprodukten von Dicarbonsäureestern mit Ethylenimin
sowie Mischungen der genannten Vernetzer und mit Epichlorhydrin beidseitig endgruppenverschlossene Polyalkylenglykole oder Blockpolymerisate aus Ethylenoxid und Propylenoxid. Besonders bevorzugt werden vernetzte, mit Ethylenimin gepfropfte Polyamidoamine auf Basis von Adipinsäure und Diethylentriamin und/oder Triethylentetramin eingesetzt. Solche Kondensationsprodukte sind beispielsweise aus der zum Stand der Technik genannten US-A-4 144 123 bekannt. Zu den besonders bevorzugten vernetzten, mit Ethylenimin gepfropften Polyamidoaminen gehören diejenigen Reaktionsprodukte, die in Gegenwart von halogenfreien Vernetzern auf Basis von Michael-Additionsprodukten von Polyetherdiaminen mit Molmassen von 400 bis 5000 oder von Polyaminen mit Molmassen von 129 bis 50000 an Acrylsäureester oder von Umsetzungsprodukten von Maleinsäureanhydrid mit Polyetherdiaminen, vgl. Formel II erhältlich sind.

Bei der Ultrafiltration werden wasserlösliche, Aminogruppen enthaltende Kondensate oder Additionsprodukte mit einer Molmassenverteilung M_{w}/Mₙ von 2 bis 350 mit einer Viskosität in 10 %iger wäßriger Lösung bei 20°C und pH 10 von mindestens 120 mPas als Retentat erhalten. Diese enger verteilten Aminogruppen enthaltenden Polymeren werden als Retentions-, Entwässerungs-, Flockungs- und Fixiermittel bei der Herstellung von Papier verwendet. Sie werden zu diesem Zweck dem Papierstoff in den üblichen Mengen von beispielsweise 0,01 bis 1 Gew.-% zugesetzt. Gegenüber der gleichen Menge an breit verteilten Aminogruppen enthaltenden Polymeren gleicher Zusammensetzung erhält man mit dem im Retentat verbleibenden Anteil an wasserlöslichen, Aminogruppen enthaltenden Kondensaten und/oder Additionsprodukten eine überraschend gesteigerte Retentions- und Entwässerungswirkung.

Ebenso sind die als Retentat anfallenden Anteile an Polymeren in Kombination mit anionischen, neutralen oder kationischen hochmolekularen Polyacrylamiden in hervorragender Weise als Retentions-, Entwässerungs- oder Fixiermittel geeignet. Ähnliche Systeme sind aus der Literatur bekannt, vgl. EP-A-0 223 223, EP-A-0 235 893 und EP-A-0 335 575.

Auch Kombinationen der als Retentat anfallenden Anteile an Polymeren mit kolloidaler Kieselsäure bzw. Bentoniten alleine oder zusätzlich noch anionischen, neutralen oder kationischen hochmolekularen Polyacrylamiden als dritter Komponente sind in ganz besonderer Weise als Retentions-, Entwässerungs- oder Fixiermittel bei der Herstellung von Papier einsetzbar. Verfahren zur Herstellung von Papier mit kationischen Hilfsmitteln, die keiner besonderen Nachbehandlung wie zum Beispiel einer Ultrafiltration unterworfen wurden, sind beispielsweise bekannt aus EP-A-0 223 223, EP-A-0 235 893 und EP-A-0 335 575. Setzt man bei den Verfahren, die z.B. aus den vorstehend genannten Literaturstellen bekannt sind, die nach dem erfindungsgemäßen Verfahren durch Ultrafiltration erhältlichen Retentate von Aminogruppen enthaltenden Kondensaten oder Additionsprodukten ein, so erhält man eine signifikante Verbesserung der Entwässerungsgeschwindigkeit und Retention bei der Papierherstellung.

Die bei der Ultrafiltration als Retentat anfallenden Anteile an Polymeren werden außerdem als Flockungsmittel für Klärschlämme, als Haftvermittler bei der Herstellung von Verbundfolien, als Additiv in Haar- und Hautpflegemitteln und als Mittel zur Immobilisierung von anionischen Wirkstoffen z.B. bei der Herstellung von Medikamenten verwendet.

Als Haftvermittler für die Herstellung von Verbundfolien werden vorzugsweise die bei der Ultrafiltration erhältlichen Retentate von Polyethyleniminen verwendet, die eine Molmasse von beispielsweise 10⁵ bis 2·10⁶ haben. Solche Retentate ergeben festere Verbunde mit einer erhöhten Alterungsbeständigkeit. Da die niedermolekularen Bestandteile aus den ultrafiltrierten Polyethyleniminen abgetrennt sind, eignen sich die Retentate insbesondere als Primer für die Herstellung von Lebensmittelverpackungen sowie als Additiv in Haar- und Hautpflegemitteln.

Die Prozentangaben in den Beispielen bedeuten Gewichtsprozent, sofern sich nichts anderes ergibt.

Die Viskositäten wurden - falls aus den Beispielen nichts anderes hervorgeht - in einem Brookfield-Viskosimeter bei 20°C, einer Konzentration von 10 Gew.-% und einem pH-Wert von 10 gemessen.

In den Beispielen, in denen eine Ultrafiltration beschrieben ist, wurden jeweils Hohlfaserkartuschen der Firma A/G-Technology Corp., Needham, MA, USA, verwendet. Die Ausschlußgrenzen (= Trenngrenzen oder Cut-off-Grenzen) der Membranen der Hohlfaserkartuschen sind in den Beispielen angegeben. Die Ultrafiltration wurde in allen Fällen beendet, sobald die Leitfähigkeit des Permeats auf Werte unterhalb von 200 µS/cm fiel.

### Beispiele

### Polymere 1a) und 1b)

### Polymer 1a)

Nach der in der DE-B-24 34 816, Beispiel 3, angegebenen Vorschrift wird durch Kondensieren von Adipinsäure mit Diethylentriamin ein Polyamidoamin hergestellt und anschließend in wäßriger Lösung mit soviel Ethylenimin gepfropft, daß das Polyamidoamin pro basischer Stickstoffgruppierung 6,7 Ethylenimin-Einheiten aufgepfropft enthält. Eine 10 %ige wäßrige Lösung des Polymeren hat eine Viskosität von 22 mPas.

### Polymer 1b)

Polymer 1a (mit Ethylenimin gepfropftes Polyamidoamin) wird durch Umsetzung mit einem Bis-Glycidylether eines Polyethylenglykols der mittleren Molmasse von 2000 gemäß den Angaben in Beispiel 3 der DE-B-24 34 816 vernetzt. Man erhält ein Ethylenimin-Einheiten enthaltendes Polymer mit einer breiten Molmassenverteilung (M_{w}/Mₙ von 400) und einer Viskosität von 120 mPas (bestimmt in 10%iger wäßriger Lösung bei 20°C und pH 10). Die Konzentration der wäßrigen Lösung beträgt 12,5 %, der pH-Wert liegt bei pH= 10.

### Polymer 2

Copolymerisat aus Acrylamid und Dimethylaminoethylacrylat, das mit Methylchlorid quaternisierc ist mit einem Gehalt an Acrylamid von 84 Mol-% und einer Molmasse von ca. 10 Mio.

### Beispiel 1

4,8 kg der oben beschriebenen 12,5 %igen wäßrigen Lösung von Polymer 1b wurden der Ultrafiltration an einer Membran mit einer Trenngrenze für Polymere mit Molmassen von 100000 (Trenngrenze = Ausschlußgrenze = Cut-off-Grenze) unterworfen. Die Membranen lagen in Form einer Hohlfaserkartusche vor. Durch Zusatz von 10 kg Wasser wurde das Volumen auf der Retentatseite aufrecht erhalten. Die Ultrafiltration wurde beendet, nachdem die Leitfähigkeit des Permeats unter 200 µS/cm lag. Auf der Retentatseite befanden sich nach Abschluß der Ultrafiltration 40 % eines wasserlöslichen, Ethylenimin-Einheiten enthaltenden Polymers 1b mit einer Molmassenverteilung M_{w}/Mₙ von 15. Die Viskosität des enger verteilten Polymeren betrug 520 mPas (gemessen in 10%iger wäßriger Lösung bei pH 10 und 20°C). 60 %, bezogen auf Polymer 1b, wurden als Permeat abgetrennt. Das Permeat wurde mit Hilfe eines Fallfilmverdampfers auf eine Konzentration von 45 % aufkonzentriert. Die Viskosität der abgetrennten niedermolekularen Anteile betrug 24 mPas (bestimmt in 10%iger wäßriger Lösung bei 20°C und pH 10).

### Beispiel 2

Beispiel 1 wurde mit der Ausnahme wiederholt, daß man unter Aufrechterhalten des Volumens auf der Retentatseite 2 kg Wasser zusetzte. Nach Abschluß der Ultrafiltration befanden sich auf der Retentatseite 60 %, bezogen auf Polymer 1b. Dieses Polymer hatte eine Molmassenverteilung M_{w}/Mₙ von 300 und eine Viskosität von 150 mPas (gemessen in 10%iger wäßriger Lösung bei 20°C). Die Polymerkonzentration des Permeats wurde durch Abdestillieren von Wasser über einen Fallfilmverdampfer auf 45 % eingestellt. Diese Lösung der niedermolekularen Polymeranteile des Permeats hatte eine Viskosität von 25 mPas.

### Beispiel 3

In einem Kolben, der mit einem Rührer und einem Rückflußkühler ausgestattet war, wurden 100 g des im Beispiel 1 abgetrennten niedermolekularen Polymeren in Form der 45 %igen wäßrigen Lösung mit 1,7 g 96 %iger Schwefelsäure versetzt und auf eine Temperatur von 85°C erwärmt. Sobald diese Temperatur erreicht war, gab man 50 g Ethylenimin als 60 %ige wäßrige Lösung zu. Im Verlauf der 6 h dauernden Reaktion fügte man 66 g Wasser zu.

### Beispiel 4

In einem mit einem Rührer und Rückflußkühler versehenen Kolben wurden 100 g der gemäß Beispiel 1 abgetrennten niedermolekularen Anteile als 45 %ige wäßrige Lösung vorgelegt, mit 1,7 g 96 %iger Schwefelsäure versetzt und auf eine Temperatur von 90°C erhitzt. Sobald diese Temperatur erreicht war, gab man 100 g Ethylenimin als 60 %ige wäßrige Lösung zu und fügte im Verlauf der Reaktion nochmals 107 g Wasser zu.

### Beispiel 5

50 g an niedermolekularem Polymer, das als Permeat im Beispiel 1 erhalten wurde, wurde als 25 %ige wäßrige Lösung in einem Kolben vorgelegt, der mit Rührer und Rückflußkühler ausgestattet war. Bei einer Temperatur von 70°C fügte man 13,5 g einer 20 %igen wäßrigen Lösung des Bis-Glycidylethers eines Polyethylenglycols der Molmasse 2000 zu. Anschließend wurde der pH-Wert des Reaktionsgemisches auf 7,8 eingestellt. Die Reaktionslösung enthielt 26,6 % Feststoffanteile und hatte eine Viskosität von 625 mPas.

### Beispiel 6

50 g des Reaktionsproduktes aus Beispiel 3 wurden als 25 %ige wäßrige Lösung in einem Reaktor vorgelegt, auf 70°C erhitzt und durch Zugabe von 12 g eines Bis-Glycidylethers von Polyethylenglycol mit einer Molmasse von 2000 in Form einer 20 %igen wäßrigen Lösung vernetzt. Der pH-Wert des Reaktionsprodukts wurde auf 7,8 eingestellt. Die wäßrige Lösung hatte einen Feststoffgehalt von 26 % und eine Viskosität von 670 mPas.

### Beispiel 7

50 g des im Beispiel 4 beschriebenen Reaktionsprodukts wurden in Form einer 25 %igen wäßrigen Lösung in einem Reaktor vorgelegt und bei einer Temperatur von 70°C mit 10 g des Bis-Glycidylethers eines Polyethylenglycols der Molmasse 2000 in Form einer 20 %igen wäßrigen Lösung vernetzt. Der pH-Wert wurde im Anschluß an die Reaktion auf 7,8 eingestellt. Man erhielt eine wäßrige Lösung mit einem Feststoffgehalt von 27,1 % und einer Viskosität von 610 mPas (bestimmt bei 20°C).

Die oben beschriebenen Produkte wurden als Entwässerungs-, Retentions- und Fixiermittel bei der Herstellung von Papier getestet. Als Maß für die Entwässerungsleistung wurde die Entwässerungszeit nach Schopper-Riegler bestimmt. Als Maß für die Retention von Faser-, Fein- und Füllstoffen wurde die optische Durchlässigkeit des ablaufenden Siebwassers herangezogen. Die Messungen erfolgten mit Hilfe eines Photometers der Firma Lange bei 588 nm.

Als Maß für die Fixierleistung von Fein- und Füllstoffen (Reinigung von belasteten geschlossenen Wasserkreisläufen) wurde die Extinktion (Lange-Photometer, 340 nm) in mit Fixiermittel behandeltem Wasser gemessen. Als Vergleich mit dem Stand der Technik wurde Polymer 1b verwendet.

Die im Folgenden angegebenen Meßergebnisse sind Mittelwerte aus 5 Messungen. Retentions- und Entwässerungsmittel wurden in Mengen von 0,01 bis 0,08 und Fixiermittel in Mengen von 0,01 bis 2 Gew.-%, jeweils bezogen auf die Feststoffe, dem Papierstoff zugesetzt.

### Beispiel 8

Aus 100 Teilen unbedrucktem Zeitungspapier mit einem Füllstoffgehalt von 10 % und 40 Teilen natürlicher Kreide (Typ DX 1 der Firma Omya) wurde eine Pulpe mit einer Stoffdichte von 5 g/l und einem Mahlgrad von 50°SR (Schopper-Riegler) hergestellt. Der pH-Wert der Pulpe betrug 7,7. Der Papierstoff wurde in mehrere Proben geteilt, die dann mit den in Tabelle 1 angegebenen Mengen an Polymeren versetzt wurden. Von jeder Probe wurden die Entwässerungszeiten für jeweils 600 ml Filtrat im Schopper-Riegler-Testgerät bestimmt.

In Abänderung der oben angegebenen Vorschrift wurde im Vergleichsbeispiel 1 die Entwässerungsgeschwindigkeit für den oben angegebenen Papierstoff in Abwesenheit eines Polymers bestimmt. Die Ergebnisse sind jeweils in Tabelle 1 angegeben.

**Tabelle 1**

| Beispiel 8 | eingesetztes Polymer | Menge an Polymer, bezogen auf trockenen Papierstoff [%] | Entwässerungszeit [sec.] |
|---|---|---|---|
| a) | Retentat von Beispiel 1 | 0,01 | 261 |
| b) | " | 0,02 | 154 |
| c) | " | 0,04 | 65 |
| d) | " | 0,08 | 28 |

| Vergl.-Beispiel | | | |
|---|---|---|---|
| 1 | - | - | 402 |
| 2a) | Polymer 1b | 0,02 | 263 |
| 2b) | " | 0,04 | 168 |
| 2c) | " | 0,08 | 89 |
| 3a) | Polymer 2 | 0,01 | 315 |
| 3b) | " | 0,02 | 218 |
| 3c) | " | 0,04 | 82 |

### Beispiel 9

Der im Beispiel 8 beschriebene Papierstoff wurde mit Ausnahme von Vergleichsbeispiel 4 mit den in Tabelle 2 angegebenen Mengen an Polymer versetzt und jeweils auf einer Versuchspapiermaschine mit einem Langsieb und angeschlossenem Akumat (Obersieb) bei einer Maschinengeschwindigkeit von 50 m/Min und einer Bahnbreite von 74 cm zu Papier mit einem Flächengewicht von 80 g/m² verarbeitet. Mit Hilfe einer automatisch arbeitenden Messanlage wurde der Füllstoffgehalt des Papiers jeweils automatisch bestimmt. Der Füllstoffgehalt des Papiers ist in Tabelle 2 jeweils angegeben. Er ist ein Maß für die Füllstoffretention.

**Tabelle 2**

| Beispiel 9 | eingesetztes Polymer | Menge an Polymer, bezogen auf trockenen Papierstoff [%] | Füllstoffgehalt des Papiers [%] |
|---|---|---|---|
| a) | Retentat von Beispiel 1 | 0,01 | 29,4 |
| b) | " | 0,02 | 34,3 |
| c) | " | 0,04 | 35,5 |
| d) | " | 0,08 | 35,7 |

| Vergl.-Beispiel | | | |
|---|---|---|---|
| 4 | - | - | 18 |
| 5a) | Polymer 1b | 0,02 | 30,2 |
| 5b) | " | 0,04 | 34,2 |
| 5c) | " | 0,08 | 35,6 |
| 6a) | Polymer 2 | 0,01 | 28,7 |
| 6b) | " | 0,02 | 32,0 |
| 6c) | " | 0,04 | 35,7 |

### Beispiel 10

Aus 100 Teilen bedrucktem Zeitungspapier wurde eine Pulpe mit einer Stoffdichte von 5 g/l und einem Mahlgrad von 50° SR hergestellt. Der pH-Wert der Pulpe betrugt 7,7. Zu Proben dieses Papierstoffs wurden mit Ausnahme von Vergleichsbeispiel 7 die in Tabelle 3 jeweils angegebenen Mengen an Polymeren zugesetzt und in einem Schopper-Riegler-Testgerät die Entwässerungszeiten für jeweils 600 ml Filtrat gemessen. Die dabei erhaltenen Ergebnisse sind in Tabelle 3 angegeben.

**Tabelle 3**

| Beispiel 10 | eingesetztes Polymer | Menge an Polymer, bezogen auf trockenen Papierstoff [%] | Entwässerungszeit [sec.] |
|---|---|---|---|
| a) | Retentat von Beispiel 1 | 0,01 | 265 |
| b) | " | 0,02 | 175 |
| c) | " | 0,04 | 76 |
| d) | " | 0,08 | 42 |

| Vergl.-Beispiel | | | |
|---|---|---|---|
| 7 | - | - | 404 |
| 8a) | Polymer 1b | 0,02 | 283 |
| 8b) | " | 0,04 | 173 |
| 8c) | " | 0,08 | 95 |
| 9a) | Polymer 2 | 0,01 | 243 |
| 9b) | " | 0,02 | 135 |

### Beispiel 11

Der im Beispiel 10 beschriebene Papierstoff wurde jeweils mit den in Tabelle 4 angegebenen Mengen an Polymeren versetzt und auf einer Versuchspapiermaschine mit einem Langsieb und angeschlossenem Akumat und einer Bahnbreite von 74 cm bei einer Maschinengeschwindigkeit von 55 m/min zu Papier mit einem Flächengewicht von 70 g/m² entwässert. Mit Hilfe einer automatisch arbeitenden Messanlage wurde der Füllstoffgehalt im Papier bestimmt, der ein Maß für die Füllstoffretention ist. Die dabei erhaltenen Ergebnisse sind in Tabelle 4 angegeben. Im Vergleichsbeispiel 10 wurde der im Beispiel 10 beschriebene Papierstoff ohne weiteren Zusatz entwässert.

**Tabelle 4**

| Beispiel 11 | eingesetztes Polymer | Menge an Polymer, bezogen auf trockenen Papierstoff [%] | Füllstoffgehalt des Papiers [%] |
|---|---|---|---|
| a) | Retentat von Beispiel 1 | 0,01 | 7,1 |
| b) | " | 0,02 | 8,3 |
| c) | " | 0,04 | 10,7 |
| d) | " | 0,08 | 10,5 |

| Vergl.-Beispiel | | | |
|---|---|---|---|
| 10 | - | - | 4,9 |
| 11a) | Polymer 1b | 0,02 | 8,0 |
| 11b) | " | 0,04 | 8,9 |
| 11c) | " | 0,08 | 9,5 |
| 12a) | Polymer 2 | 0,01 | 7,5 |
| 12b) | " | 0,02 | 8,2 |
| 12c) | " | 0,04 | 9,2 |

### Beispiel 12

Aus 50 Teilen gebleichtem Kiefernsulfatzellstoff, 50 Teilen gebleichtem Buchensulfitzellstoff und 30 Teilen einer natürlichen Kreide (Typ DX 1 der Firma Omya) wurde eine Pulpe mit einer Stoffdichte von 5 g/l und einem Mahlgrad von 27° SR hergestellt. Der pH-Wert der Pulpe betrug 7,2. Mit Ausnahme von Vergleichsbeispiel 13 wurden zu Proben dieses Papierstoffs die in Tabelle 5 angegebenen Mengen an Polymer zugegeben und jeweils die Entwässerungszeiten für 600 ml Filtrat in einem Schopper-Riegler-Testgerät bestimmt. Die Entwässerungszeiten sind in Tabelle 5 angegeben.

**Tabelle 5**

| Beispiel 12 | eingesetztes Polymer | Menge an Polymer, bezogen auf trockenen Papierstoff [%] | Entwässerungszeit [sec.] |
|---|---|---|---|
| a) | Retentat von Beispiel 1 | 0,02 | 60 |
| b) | " | 0,04 | 35 |

| Vergl.-Beispiel | | | |
|---|---|---|---|
| 13 | - | - | 114 |
| 14a) | Polymer 1b | 0,02 | 96 |
| 14b) | " | 0,04 | 65 |
| 15 | Polymer 2 | 0,02 | 90 |

### Beispiel 13

Aus dem im Beispiel 12 beschriebenen Papierstoff wurden mit Ausnahme von Vergleichsbeispiel 16 die in Tabelle 6 angegebenen Mengen an Polymer zugesetzt. Auf einer Versuchspapiermaschine mit einem Langsieb und angeschlossenem Akumat (Obersieb) und einer Bahnbreite von 74 cm wurden bei unterschiedlichen Maschinengeschwindigkeiten Papier mit einem Flächengewicht von 70 g/m² hergestellt. Der Füllstoffgehalt der Papiere wurde mit Hilfe einer automatisch arbeitenden Meßanlage bestimmt. Die Versuchsbedingungen und Ergebnisse sind in Tabelle 6 angegeben.

**Tabelle 6**

| Beispiel 13 | eingesetztes Polymer | Menge an Polymer, bezogen auf trokkenen Papierstoff [%] | Maschinengeschwindigkeit [m/min] | Füllstoffgehalt des Papiers [%] |
|---|---|---|---|---|
| a) | Retentat von Beispiel 1 | 0,02 | 50 | 27,7 |
| b) | " | 0,04 | 52 | 29,1 |

| Vergl.-Beispiel | | | | |
|---|---|---|---|---|
| 16 | - | - | 40 | 11,2 |
| 17a) | Polymer 1b | 0,02 | 46 | 25,4 |
| 17b) | " | 0,04 | 48 | 27,5 |
| 18a) | Polymer 2 | 0,01 | 48 | 18,2 |
| 18b) | " | 0,02 | 50 | 24,8 |

### Beispiel 14

Aus 100 Teilen unbedrucktem Zeitungspapier mit einem Füllstoffgehalt von ca. 10 % und 10 Teilen China Clay (Type X 1 der Firma ECC) wurde eine Pulpe mit einer Stoffdichte von 5 g/l und einem Mahlgrad von 50° SR hergestellt. Der pH-Wert betrug 7,6. Mit Ausnahme von Vergleichsbeispiel 19 wurden zu diesem Papierstoff jeweils die in Tabelle 7 angegebenen Mengen an Polymer zugegeben und die Entwässerungszeiten der so erhaltenen Papierstoffe in einem Schopper-Riegler-Testgerät und die optische Durchlässigkeit des Siebwassers bestimmt. Die Ergebnisse sind in Tabelle 7 angegeben.

**Tabelle 7**

| Beispiel 14 | eingesetztes Polymer | Menge an Polymer, bezogen auf trokkenen Papierstoff [%] | Entwässerungszeit [sec.] | Optische Durchlässigkeit [%] |
|---|---|---|---|---|
| a) | Retentat von Beispiel 1 | 0,02 | 41 | 64 |
| b) | " | 0,04 | 27 | 79 |
| c) | " | 0,06 | 21 | 80 |
| b) | " | 0,08 | 19 | 84 |

| Vergl.-Beispiel | | | | |
|---|---|---|---|---|
| 19 | - | - | 92 | 23 |
| 20a) | Polymer 1b | 0,02 | 58 | 52 |
| 20b) | " | 0,04 | 43 | 65 |
| 20c) | " | 0,06 | 34 | 71 |
| 20d) | " | 0,08 | 30 | 74 |

### Beispiele 15 bis 17

Aus 100 Teilen unbedrucktem Zeitungspapier mit einem Füllstoffgehalt von 10 % und 10 Teilen China Clay der Firma ECC, Type X 1, wird eine Pulpe mit einer Stoffdichte von 2,2 g/l und einem Mahlgrad von 50° SR hergestellt. Der pH-Wert der Pulpe betrug 7. Mit Ausnahme von Vergleichsbeispiel 21 wird zu Proben dieses Papierstoffs jeweils das in Tabelle 8 jeweils angegebene Polymer in der dort bezeichneten Menge zugesetzt. Nach dem Durchmischen werden die Proben in einem Schopper-Riegler-Testgerät jeweils entwässert und die Entwässerungszeit für 600 ml Filtrat bestimmt. In Tabelle 8 ist außerdem die optische Durchlässigkeit des Filtrats angegeben.

**Tabelle 8**

| Beispiel | eingesetztes Polymer | Menge an Polymer, bezogen auf trokkenen Papierstoff [%] | Entwässerungszeit [sec.] | Optische Durchlässigkeit [%] |
|---|---|---|---|---|
| 15a) | Polymer hergestellt nach Beispiel 5 | 0,02 | 55 | 48 |
| 15b) | " | 0,04 | 43 | 62 |
| 15c) | " | 0,06 | 36 | 69 |
| 15d) | " | 0,08 | 29 | 75 |
| 16a) | Polymer hergestellt nach Beispiel 6 | 0,02 | 54 | 51 |
| 16b) | " | 0,04 | 38 | 66 |
| 16c) | " | 0,06 | 30 | 74 |
| 16d) | " | 0,08 | 26 | 76 |
| 17a) | Polymer hergestellt nach Beispiel 7 | 0,02 | 54 | 48 |
| 17b) | " | 0,04 | 38 | 66 |
| 17c) | " | 0,06 | 29 | 73 |
| 17d) | " | 0,08 | 25 | 77 |

| Vergl. Beispiel | | | | |
|---|---|---|---|---|
| 21a) | Polymer 1b | 0,02 | 53 | 52 |
| 21b) | " | 0,04 | 38 | 65 |
| 21c) | " | 0,06 | 31 | 76 |
| 21d) | " | 0,08 | 26 | 78 |
| 22 | - | - | 84 | 26 |

### Beispiele 18 bis 20

Aus 100 Teilen unbedrucktem Zeitungspapier mit einem Füllstoffgehalt von ca. 10 % und 10 Teilen China Clay der Firma ECC, Type X 1, sowie 0,5 % Alaun, bezogen auf trockenen Stoff, wurde eine Pulpe mit einer Stoffdichte von 2,2 g/l und einem Mahlgrad von 50° SR hergestellt. Der pH-Wert der Pulpe betrug 6,0. Mit Ausnahme von Vergleichsbeispiel 23 wurden zu Proben dieses Papierstoffs die in Tabelle 9 jeweils angegebenen Mengen an Polymerisaten zugesetzt und die Entwässerungszeit für 600 ml Filtrat in einem Schopper-Riegler-Testgerät bestimmt. Außerdem ist in Tabelle 9 die optische Durchlässigkeit des Filtrats angegeben.

**Tabelle 9**

| Beispiel | eingesetztes Polymer | Menge an Polymer, bezogen auf trokkenen Papierstoff [%] | Entwässerungszeit [sec.] | Optische Durchlässigkeit [%] |
|---|---|---|---|---|
| 18a) | Polymer hergestellt nach Beispiel 5 | 0,02 | 47 | 56 |
| 18b) | " | 0,04 | 37 | 63 |
| 18c) | " | 0,06 | 33 | 68 |
| 18d) | " | 0,08 | 30 | 71 |
| 19a) | Polymer hergestellt nach Beispiel 6 | 0,02 | 47 | 58 |
| 19b) | " | 0,04 | 34 | 69 |
| 19c) | " | 0,06 | 29 | 75 |
| 19d) | " | 0,08 | 28 | 76 |
| 20a) | Polymer hergestellt nach Beispiel 7 | 0,02 | 45 | 60 |
| 20b) | " | 0,04 | 33 | 68 |
| 20c) | " | 0,06 | 29 | 74 |
| 20d) | " | 0,08 | 26 | 77 |

| Vergl.-Beispiel | | | | |
|---|---|---|---|---|
| 23 | - | - | 77 | 30 |
| 24a) | Polymer 1b | 0,02 | 44 | 59 |
| 24b) | " | 0,04 | 34 | 62 |
| 24c) | " | 0,06 | 29 | 74 |
| 24d) | " | 0,08 | 27 | 76 |

### Beispiele 21 bis 23

Aus 33 Teilen unbedrucktem Zeitungspapier, 33 Teilen Wellpappe und 33 Teilen bedrucktem LWC-Papier wird eine Pulpe mit einer Stoffdichte von 2 g/l und einem Mahlgrad von 50° SR hergestellt. Der pH-Wert der Pulpe betrug 7. Mit Ausnahme von Vergleichsbeispiel 25 wurden zu Proben dieses Papierstoffs die in Tabelle 10 jeweils angegebenen Mengen an Polymeren zugesetzt und die Entwässerungszeiten für 600 ml Filtrat in einem Schopper-Riegler-Testgerät bestimmt. Außerdem wurde die optische Durchlässigkeit des Filtrats gemessen. Die Ergebnisse sind in Tabelle 10 angegeben.

**Tabelle 10**

| Beispiel | eingesetztes Polymer | Menge an Polymer, bezogen auf trokkenen Papierstoff [%] | Entwasserungszeit [sec.] | Optische Durchlässigkeit [%] |
|---|---|---|---|---|
| 21a) | Polymer hergestellt nach Beispiel 5 | 0,02 | 61 | 44 |
| 21b) | " | 0,04 | 49 | 56 |
| 21c) | " | 0,06 | 41 | 62 |
| 21d) | " | 0,08 | 36 | 67 |
| 22a) | Polymer hergestellt nach Beispiel 6 | 0,02 | 57 | 50 |
| 22b) | " | 0,04 | 42 | 58 |
| 22c) | " | 0,06 | 35 | 67 |
| 22d) | " | 0,08 | 28 | 71 |
| 23a) | Polymer hergestellt nach Beispiel 7 | 0,02 | 59 | 43 |
| 23b) | " | 0,04 | 42 | 62 |
| 23c) | " | 0,06 | 35 | 68 |
| 23d) | " | 0,08 | 29 | 72 |

| Vergl.-Beispiel | | | | |
|---|---|---|---|---|
| 25 | - | - | 82 | 27 |
| 26a) | Polymer 1b | 0,02 | 59 | 45 |
| 26b) | " | 0,04 | 43 | 60 |
| 26c) | " | 0,06 | 35 | 67 |
| 26d) | " | 0,08 | 31 | 69 |

### Beispiele 24 bis 26

Aus 33 Teilen unbedrucktem Zeitungspapier, 33 Teilen Wellpappe und 33 Teilen bedrucktem LWC-Papier wird eine Pulpe mit einer Stoffdichte von 2 g/l und einem Mahlgrad von 50° SR hergestellt. Der pH-Wert der Pulpe betrug 7. Dieser Pulpe setzte man 0,2 % Wasserglas (fest), bezogen auf trockenen Papierstoff zu. Mit Ausnahme von Vergleichsbeispiel 27 setzte man zu Proben dieses Papierstoffs jeweils die in Tabelle 11 angegebenen Mengen an Polymer zu und bestimmte für diese Proben die Entwässerungszeiten in einem Schopper-Riegler-Testgerät für 600 ml Filtrat. Außerdem wurde die optische Durchlässigkeit der Filtrate gemessen. Die Ergebnisse sind in Tabelle 11 angegeben.

**Tabelle 11**

| Beispiel | eingesetztes Polymer | Menge an Polymer, bezogen auf trokkenen Papierstoff [%] | Entwässerungszeit [sec.] | Optische-Durchlässigkeit [%] |
|---|---|---|---|---|
| 24a) | Polymer hergestellt nach Beispiel 5 | 0,02 | 82 | 21 |
| 24b) | " | 0,04 | 74 | 27 |
| 24c) | " | 0,06 | 69 | 35 |
| 24d) | " | 0,08 | 62 | 44 |
| 25a) | Polymer hergestellt nach Beispiel 6 | 0,02 | 82 | 25 |
| 25b) | " | 0,04 | 69 | 33 |
| 25c) | " | 0,06 | 52 | 52 |
| 25d) | " | 0,08 | 42 | 67 |
| 26a) | Polymer hergestellt nach Beispiel 7 | 0,02 | 84 | 23 |
| 26b) | " | 0,04 | 70 | 34 |
| 26c) | " | 0,06 | 56 | 52 |
| 26d) | " | 0,08 | 50 | 58 |

| Vergl. Beispiel | eingesetztes Polymer | Menge an Polymer, bezogen auf trokkenen Papierstoff [%] | Entwässerungszeit [sec.] | Optische Durchlässigkeit [%] |
|---|---|---|---|---|
| 27 | - | - | 82 | 20 |
| 28a) | Polymer 1b | 0,02 | 75 | 21 |
| 28b) | " | 0,04 | 71 | 27 |
| 28c) | " | 0,06 | 67 | 41 |
| 28d) | " | 0,08 | 56 | 51 |

### Beispiel 27

4 kg einer 20 %igen wäßrigen Lösung von Polymer 1a) wurden unter Aufrechterhalten des Volumens auf der Retentatseite durch Zusatz von 12 kg Wasser über eine Hohlfaserkartusche der Firma A/G-Technology mit einer Ausschlußgrenze von 3000 filtriert, bis die Leitfähigkeit des Permeats unterhalb von 200 µS/cm lag. Auf der Retentatseite befanden sich 18,2 % des eingesetzten Polymeren. Das Permeat wurde mit Hilfe eines Fallfilmverdampfers auf eine Konzentration von 62,5 % eingedampft. Ausgangspolymer, Retentat und Filtrat hatten folgende Eigenschaften:

| | Viskosität [mPas] | M_{w}/Mₙ |
|---|---|---|
| | (10 %ige wäßr. Lösung, pH 10, 20°C) | |
| Polymer 1a) | 22 | 5,3 |
| Retentat | 36 | 2,7 |
| Permeat | 20 | 4,5 |

600 g einer 25 %igen wäßrigen Lösung des oben beschriebenen Retentats wurden in einem mit einem Rührer ausgestatteten Reaktor bei einer Temperatur von 70°C mit 55 g einer 20 %igen wäßrigen Lösung eines Bisglycidylether eines Polyethylenglykols der Molmasse 1500 vernetzt. Man erhielt eine 25,8 %ige wäßrige Lösung. Durch Zugabe von Schwefelsäure wurde der pH-Wert auf 7,9 eingestellt. Eine 10 %ige wäßrige Lösung hatte eine Viskosität von 1170 mPas.

### Beispiel 28

600 g einer 25 %igen wäßrigen Lösung des Permeats, das im Beispiel 27 beschrieben ist, wurden in einem mit einem Rührer ausgestatteten Reaktor vorgelegt und bei einer Temperatur von 70°C durch Zugabe von 55 g einer 20 %igen wäßrigen Lösung des Bis-glycidylethers vom Polyethylenglykol einer Molmasse von 1500 vernetzt. Man erhielt eine wäßrige Polymerlösung mit einem Feststoffgehalt von 24,9 %. Der pH-Wert der Lösung wurde durch Zugabe von Schwefelsäure auf 7,8 eingestellt. Eine 10 %ige wäßrige Lösung hatte eine Viskosität von 950 mPas.

### Beispiel 29

4,8 kg einer 12,5 %igen wäßrigen Lösung von Polymer 1b wurden einer Ultrafiltration über eine Hohlfaserkartusche der Fa. A/G-Technology Corp. mit einer Ausschlußgrenze von 3000 unterworfen. Durch Zusatz von Wasser wurde das Volumen auf der Retentatseite aufrechterhalten. Man erhielt 11,4 kg eines Filtrats (Permeat), das einen Feststoffgehalt von 2,4 % hatte. Hierbei wurden 44 % des Polymeren abgetrennt.

### Beispiel 30

Das Retentat von Beispiel 29 wurde der Ultrafiltration über eine Hohlfaserkartusche mit einer Ausschlußgrenze von 10000 unterworfen, wobei man das Volumen auf der Seite des Retentats durch Wasserzusatz aufrechterhielt. Die Menge des Filtrats (Permeat) betrug 6,1 kg, der Feststoffgehalt des Permeats 0,4 %. 8 % Polymer wurden abgetrennt.

### Beispiel 31

Das Retentat von Beispiel 30 wurde der Ultrafiltration in einer Hohlfaserkartusche mit einer Ausschlußgrenze von 30000 unterworfen. Durch Zusatz von Wasser wurde das Volumen auf der Retentatseite aufrechterhalten. Man erhielt 7,1 kg eines Filtrats (Permeat) mit einem Feststoffgehalt von 0,5 %. Mann trennte 13 % Polymer ab.

### Beispiel 32

Das Retentat von Beispiel 31 wurde unter Aufrechterhaltung des Volumens auf der Retentatseite durch Wasserzusatz über eine Hohlfaserkartusche mit einer Ausschlußgrenze von 100000 ultrafiltriert. Man erhielt 8,7 kg Filtrat (Permeat) mit einem Feststoffgehalt von 0,7 %. Man trennte 25 % Polymer ab.

### Beispiel 33

Das Retentat von Beispiel 32 wurde unter Aufrechterhalten des Volumens auf der Retentatseite durch Wasserzusatz über eine Hohlfaserkartusche mit einer Ausschlußgrenze von 300000 ultrafiltriert. Man erhielt 6 kg Filtrat (Permeat) mit einem Feststoffgehalt von 0,3 %. Der Anteil des als Permeat abgetrennten Polymeren betrug 9 %.

### Beispiel 34

Das nach Beispiel 33 erhaltene Retentat wurde unter Aufrechterhaltung des Vclumens auf der Retentatseite durch Wasserzusatz über eine Hohlfaserkartusche mit einer Ausschlußgrenze von 500000 ultrafiltriert. Man erhielt 6,4 kg Filtrat (Permeat) das einen Feststoffgehalt von 0,3 % hatte. Der Anteil des als Permeat abgetrennten Polymeren betrug 12 %.

### Beispiel 35

Das nach Beispiel 34 erhaltene Retentat wurde mit Hilfe fortgesetzter Ultrafiltration über eine Hohlfaserkartusche mit einer Ausschlußgrenze von 500000 bis auf ein Gewicht von 2,1 kg mit einem Feststoffgehalt von 7,1 % konzentriert.

Die nach den Beispielen 29 bis 35 durch Ultrafiltration abgetrennten Polymeren wurden in den folgenden Beispielen als Entwässerungs-, Retentions- und Fixiermittel bei der Herstellung von Papier getestet.

### Beispiele 36 und 37

Aus 100 Teilen unbedrucktem Zeitungspapier mit einem Füllstoffgehalt von ca. 10 % und 10 Teilen China Clay des Typs X1 der Firma ECC wurde eine Pulpe mit einer Stoffdiche von 2,2 g/l und einem Mahlgrad von 61°SR hergestellt. Der pH-Wert der Pulpe lag bei 7,5. Zu Proben dieses Papierstoffs wurden die in Tabelle 12 angegebenen Mengen an Polymeren, bezogen auf trockenen Papierstoff, zugegeben und mit dem Papierstoff durchmischt. Unmittelbar danach wurden die Entwässerungszeiten für jeweils 600 ml Filtrat in einem Schopper-Riegler-Testgerät bestimmt. Dabei wurden die in Tabelle 12 angegebenen Ergebnisse erhalten. Zum Vergleich wurde die Blattbildung ohne einen Polymerzusatz und mit einem Zusatz von Polymer 1b geprüft.

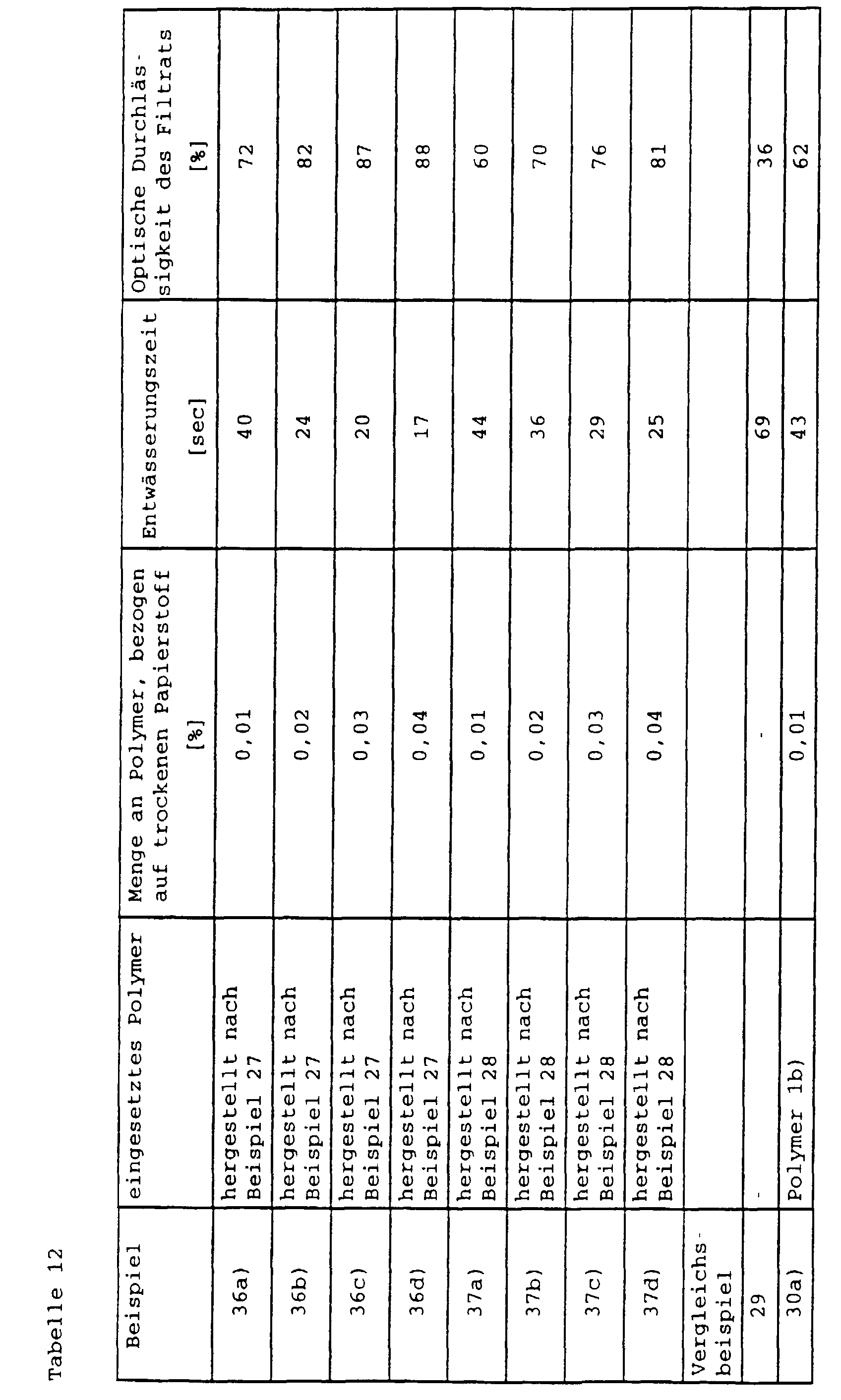

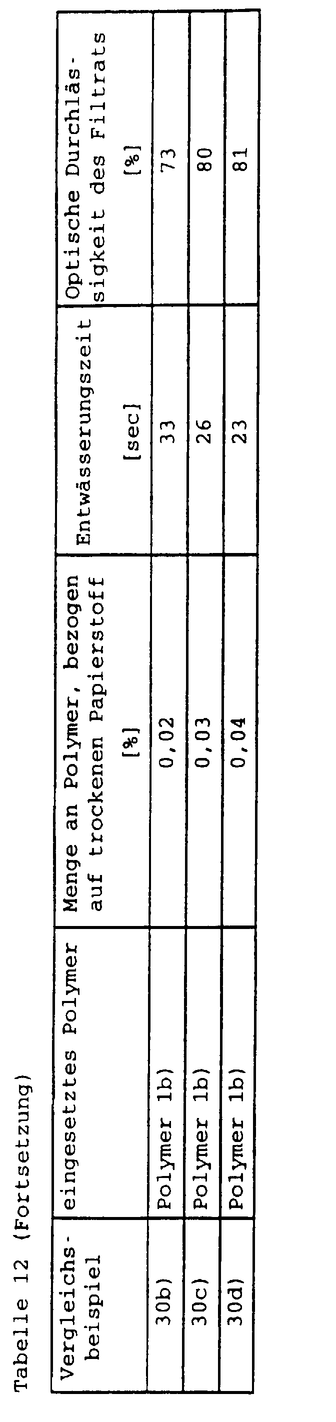

### Beispiele 38 bis 44

Aus 100 Teilen unbedrucktem Zeitungspapier mit einem Füllstoffgehalt von ca. 10 % und 20 Teilen natürlicher Kreide des Typs DX1 der Firma Omya wurde eine Pulpe mit einer Stoffdichte von 2,4 g/l und einem Mahlgrad von 61°SR hergestellt. Der pH-Wert der Pulpe lag bei 7,5. Zu Proben dieses Papierstoffs wurden jeweils die in Tabelle 13 angegebenen Polymeren zugesetzt und mit dem Papierstoff vermischt. Die so erhaltenen Mischungen wurden anschließend jeweils in einem Schopper-Riegler-Testgerät entwässert. Man bestimmte die Entwässerungszeiten für jeweils 600 ml Filtrat. Außerdem wurde die optische Durchlässigkeit des Filtrats gemessen. Die Versuchsbedingungen und Ergebnisse sind in Tabelle 13 angegeben.

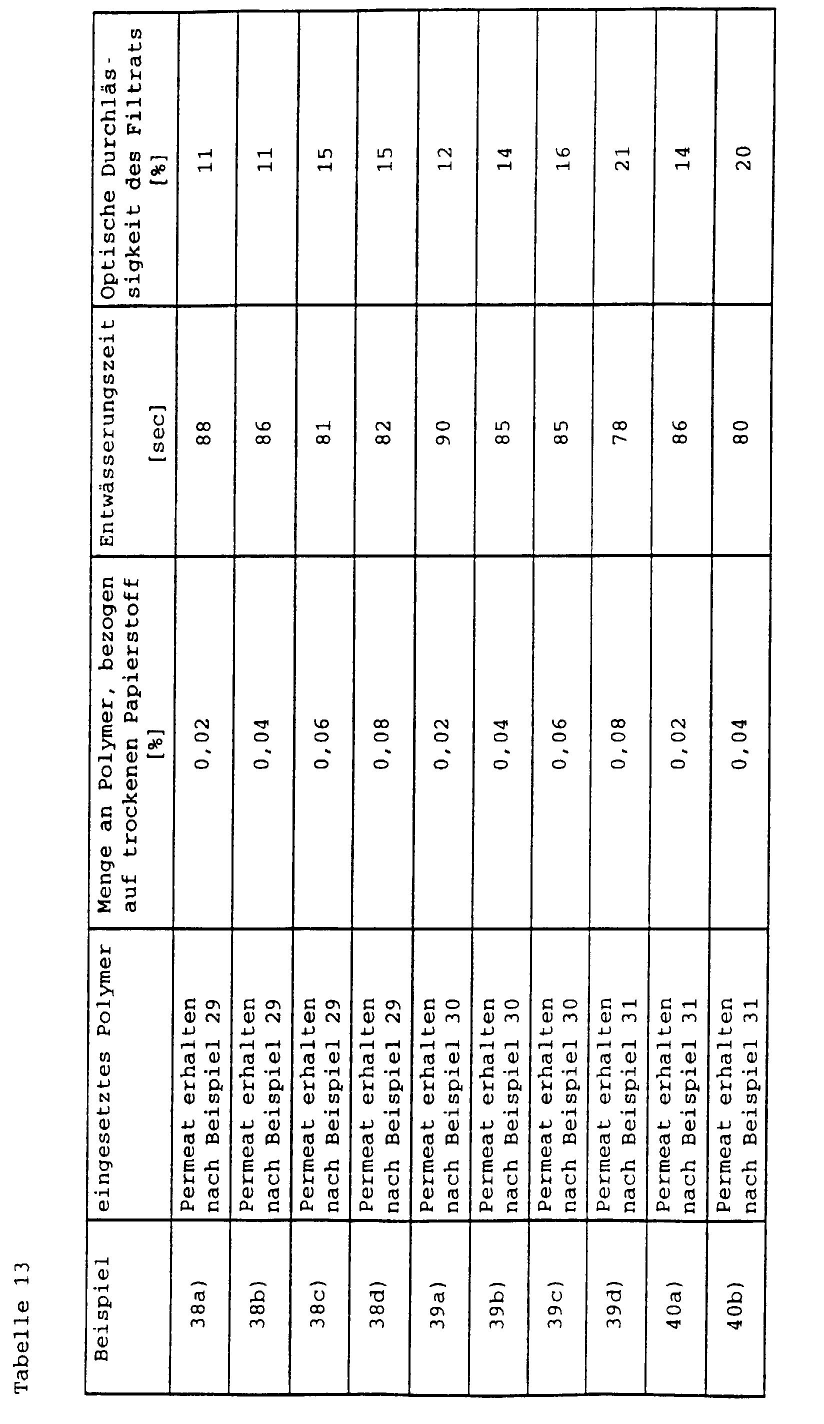

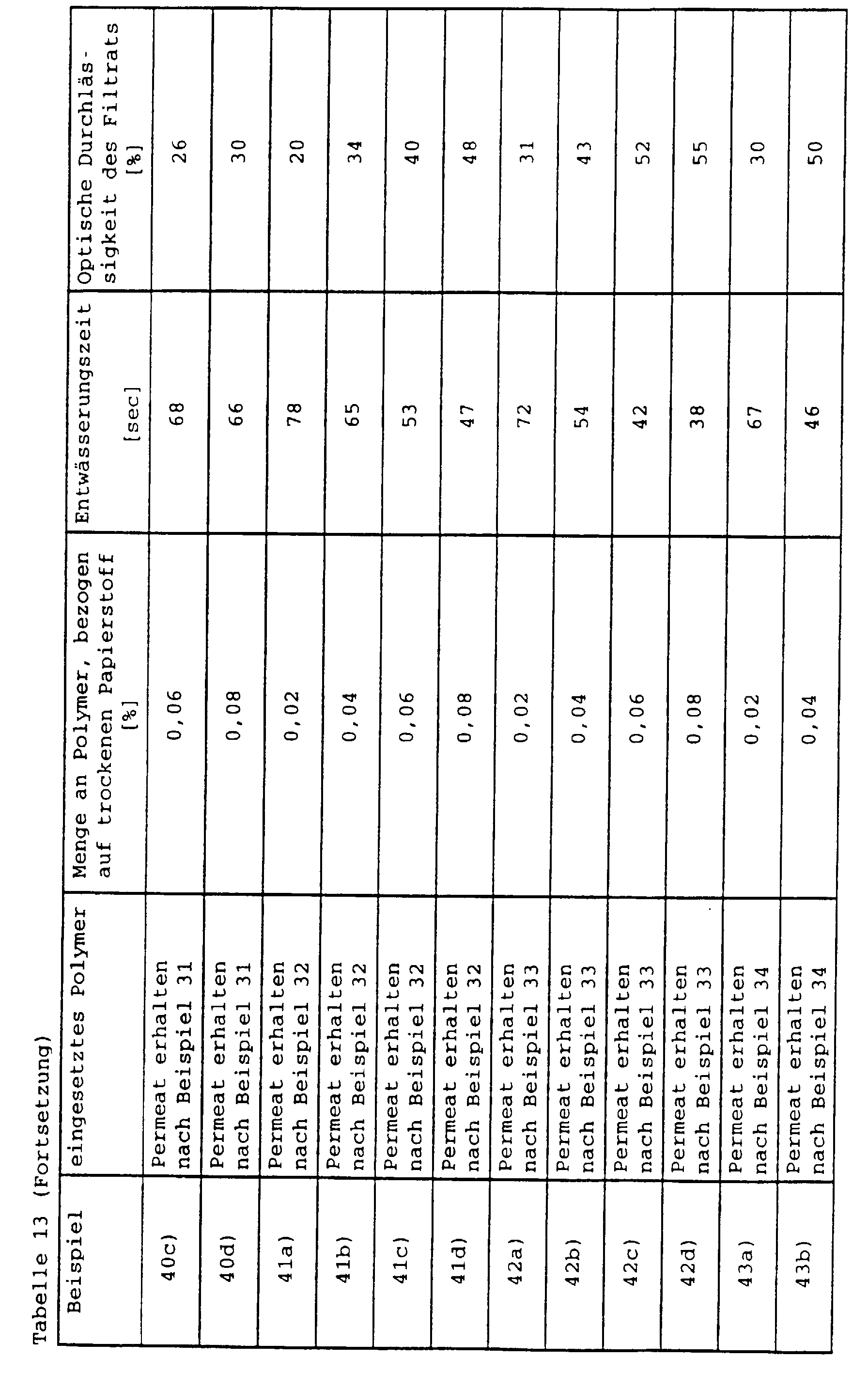

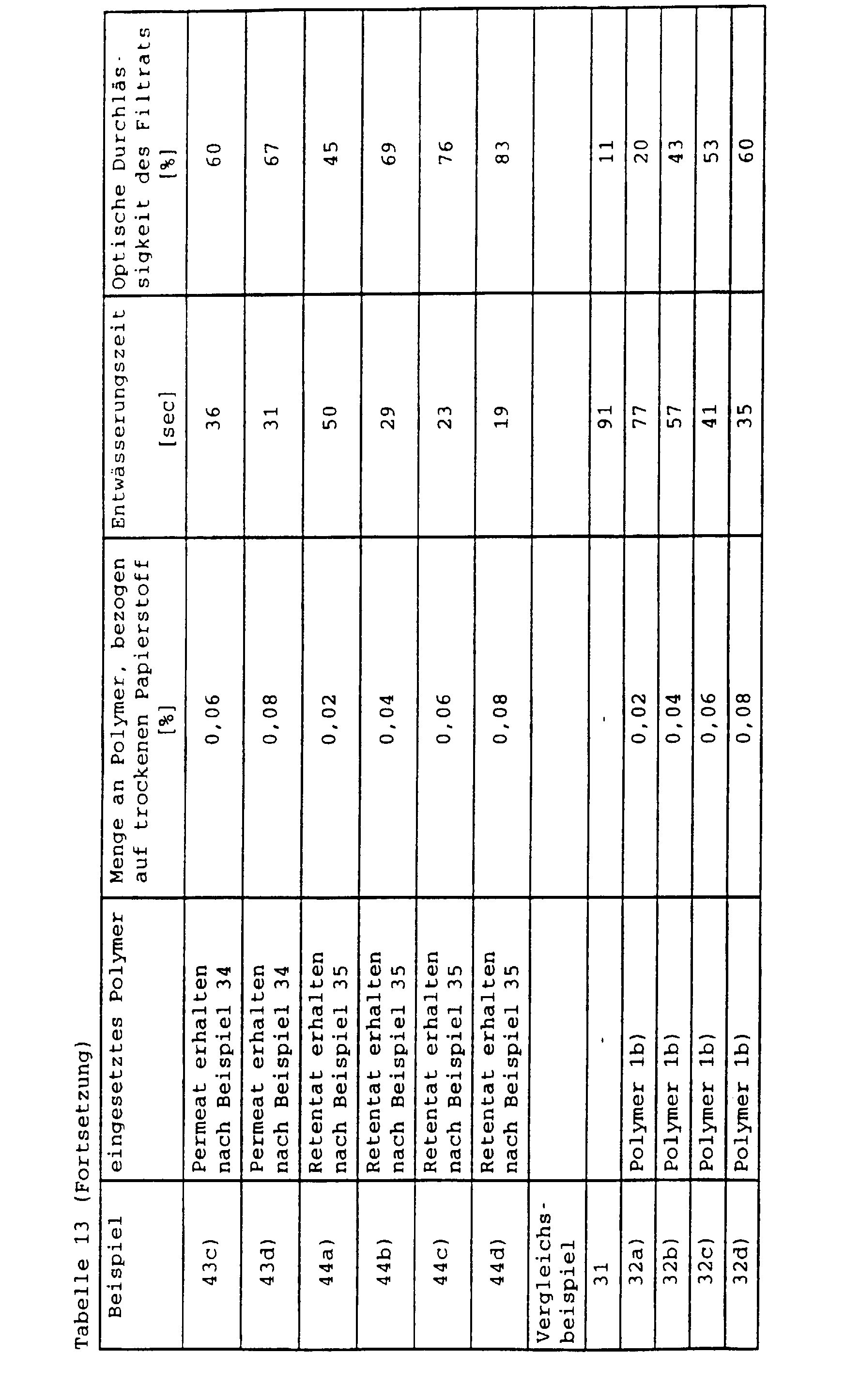

### Beispiele 45 bis 51

Aus einer Mischung aus 33 Teilen unbedrucktem Zeitungspapier, 33 Teilen Wellpappe und 33 Teilen bedrucktem LWC-Papier vom Mahlgrad 58°C SR wurde eine Pulpe mit einer Stoffdichte von 2 g/l hergestellt. Der pH-Wert der Pulpe betrug 7,5. Zu Proben dieses Papierstoffs wurden die in Tabelle 14 angegebenen Polymerisate zugesetzt und nach dem Mischen jeweils in einem Schopper-Riegler-Testgerät entwässert. Man bestimmte die Entwässerungszeiten für jeweils 600 ml Filtrat sowie die optische Durchlässigkeit des Filtrats. Die Ergebnisse sind in der Tabelle 14 zusammen mit den Ergebnissen von Vergleichsbeispielen angegeben.

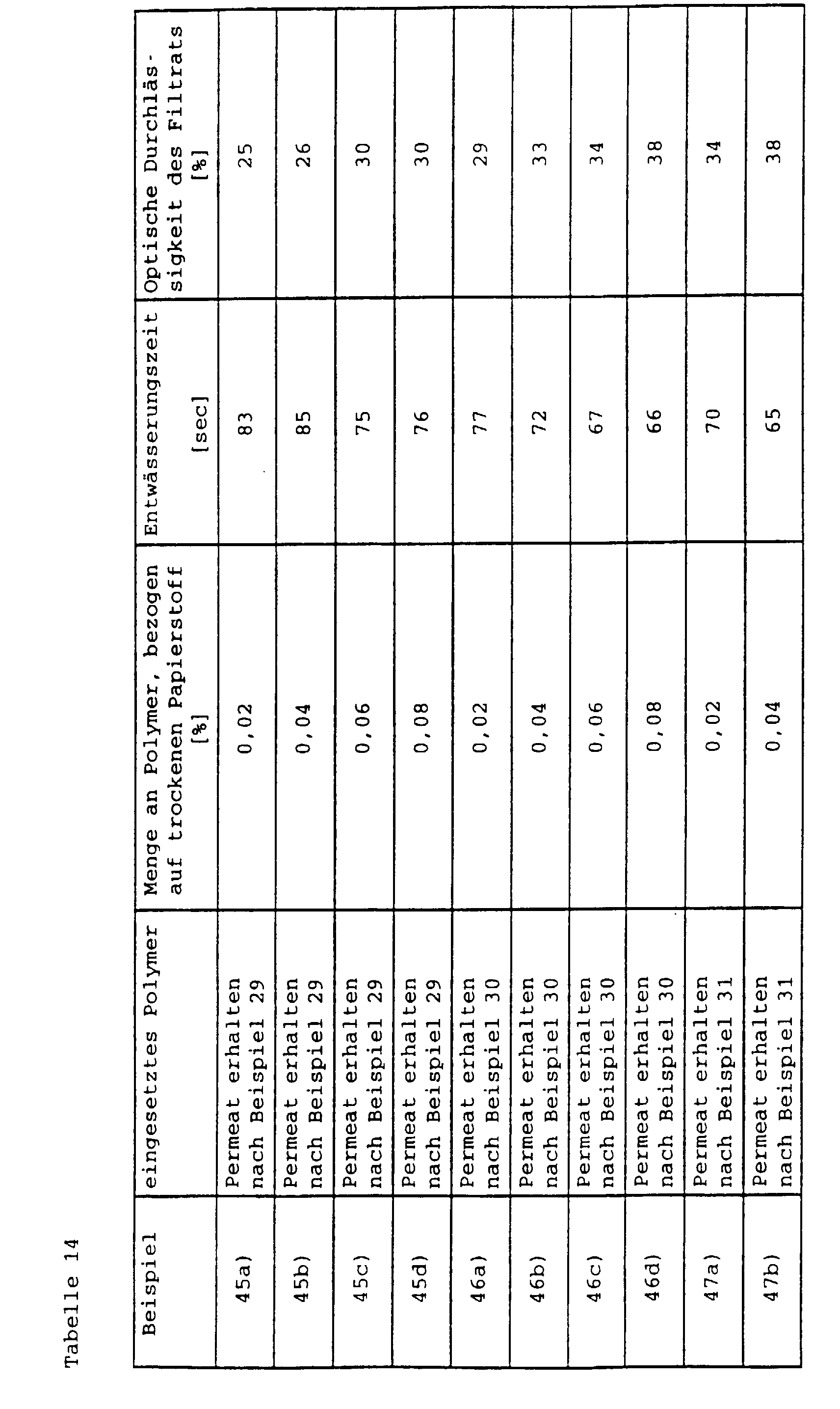

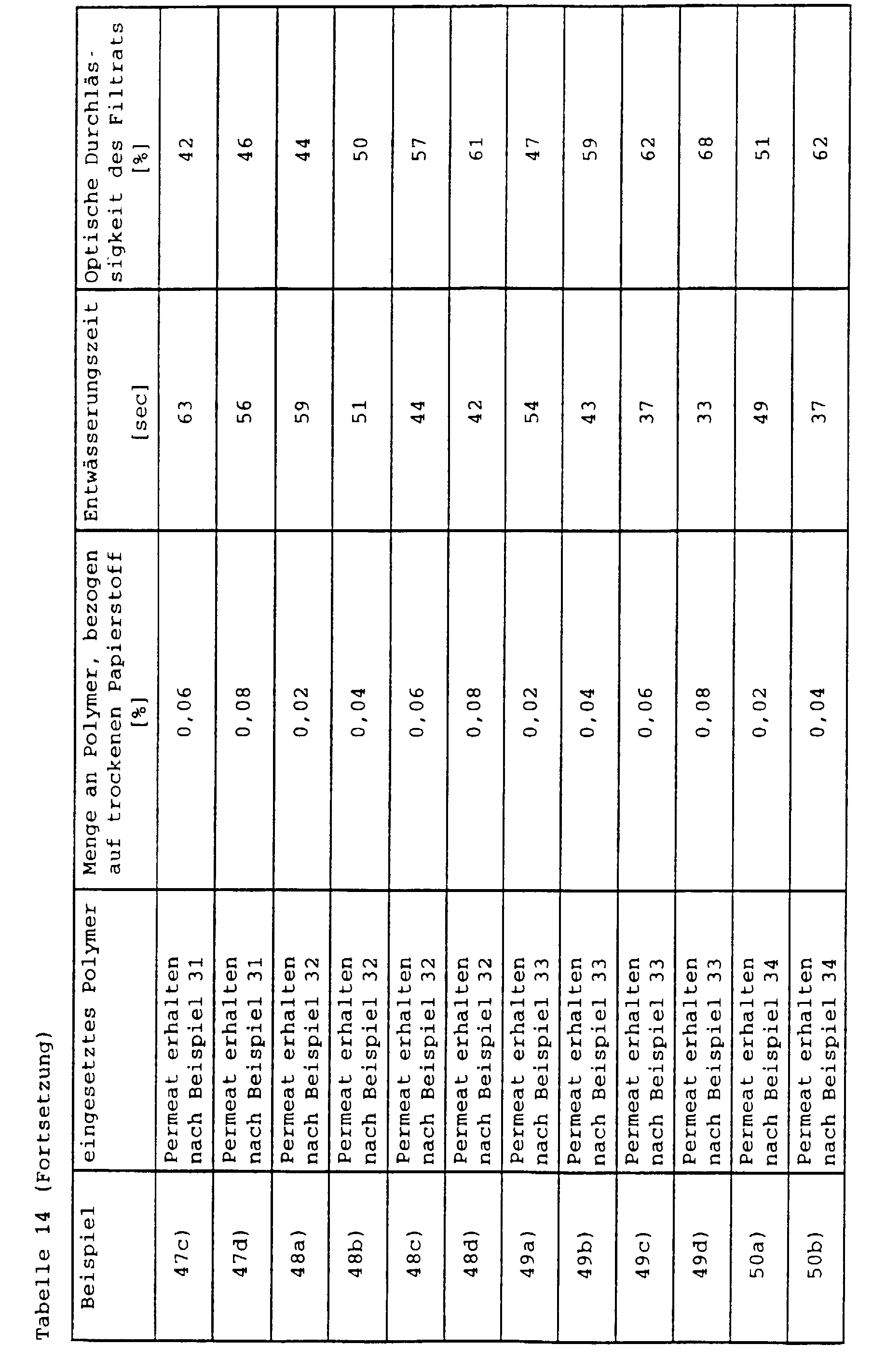

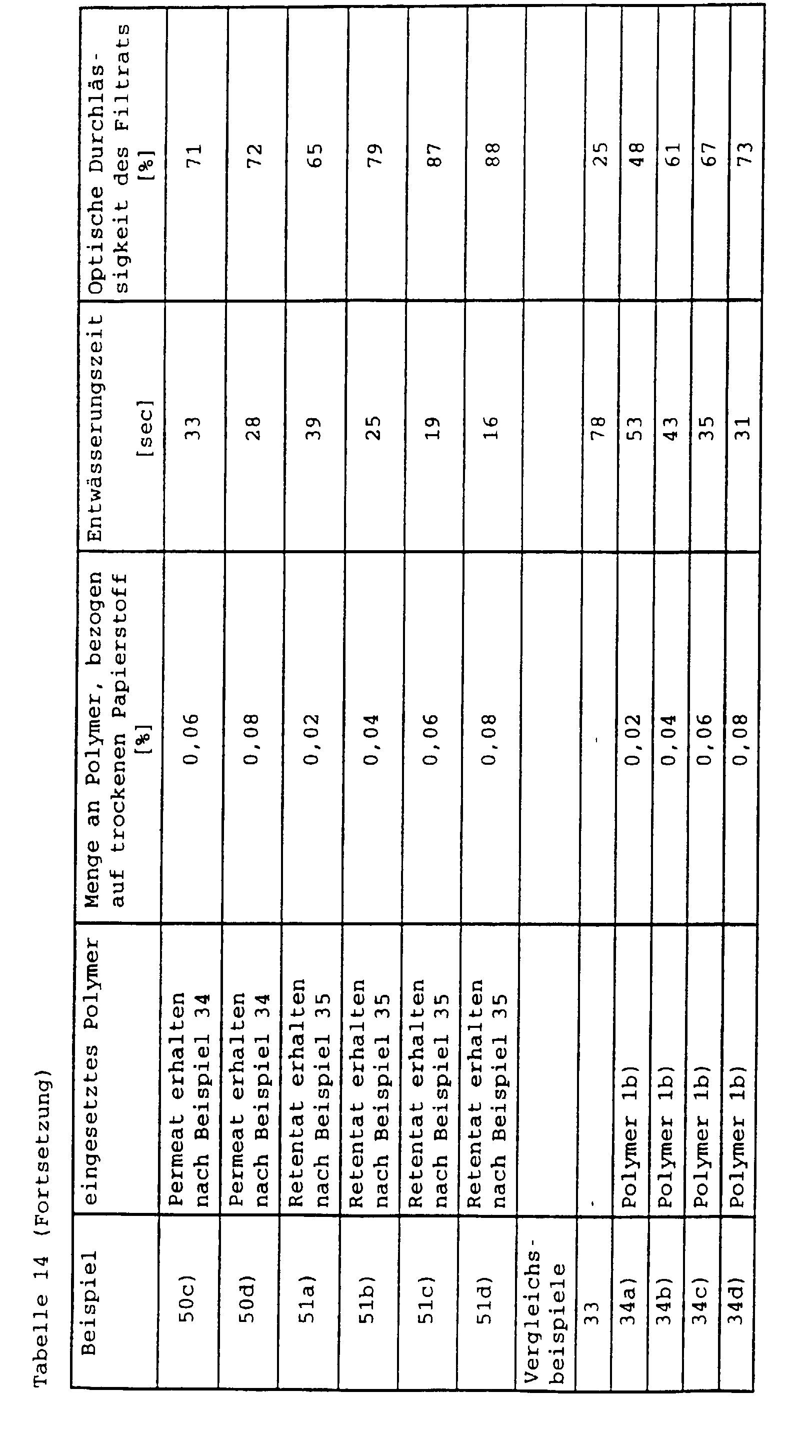

### Beispiele 52 - 58

Aus TMP (thermomechanische Pulpe) wurde eine wäßrige Faseraufschlammung mit einer Stoffkonzentration von 2 % hergestellt und mit einer wäßrigen Lösung von Huminsäure als Störstoff versetzt. Die so hergestellte Pulpe diente als Prüfsubstanz. Zu Proben dieser Pulpe gab man jeweils die in Tabelle 15 angegebenen Mengen an Polymer und zusätzlich noch jeweils die gleiche Menge eines kationischen Polyacrylamids als Flockungsmittel. Die Proben wurden geschüttelt und anschließend filtriert. Um die Fixierwirkung der in Tabelle 15 angegebenen Polymeren zu beurteilen, wurde die Extinktion der jeweils erhaltenen Filtrate bestimmt. Die Meßwerte sind in Tabelle 15 angegeben. Wie daraus hervorgeht, sind Polymere mit niedriger Molmasse kaum wirksam. Dagegen zeigen mittel- und hochmolekulare Fraktionen zum Teil bessere Wirksamkeiten als der Vergleich Polymer 1b). Beispiele 55, 56 und 58 ergaben bessere Fixierwirkung als Polymer 1b).

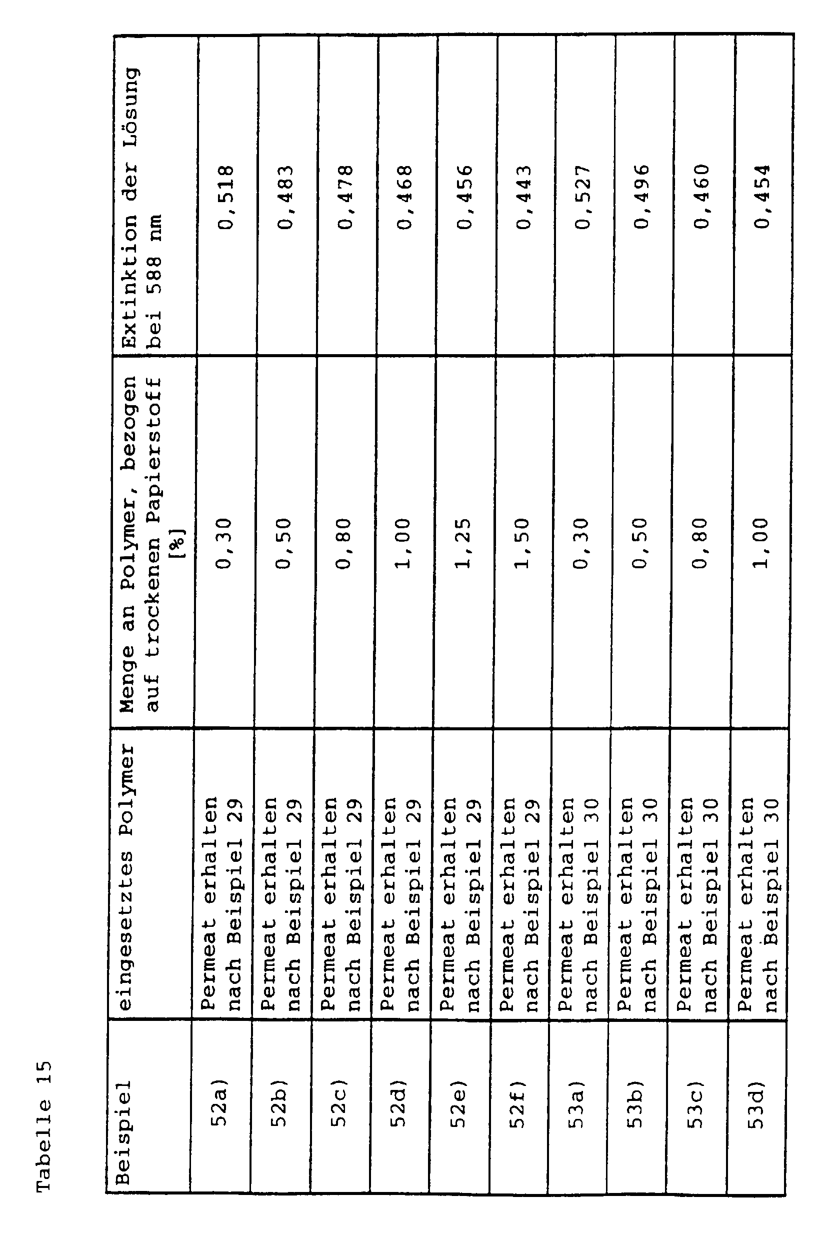

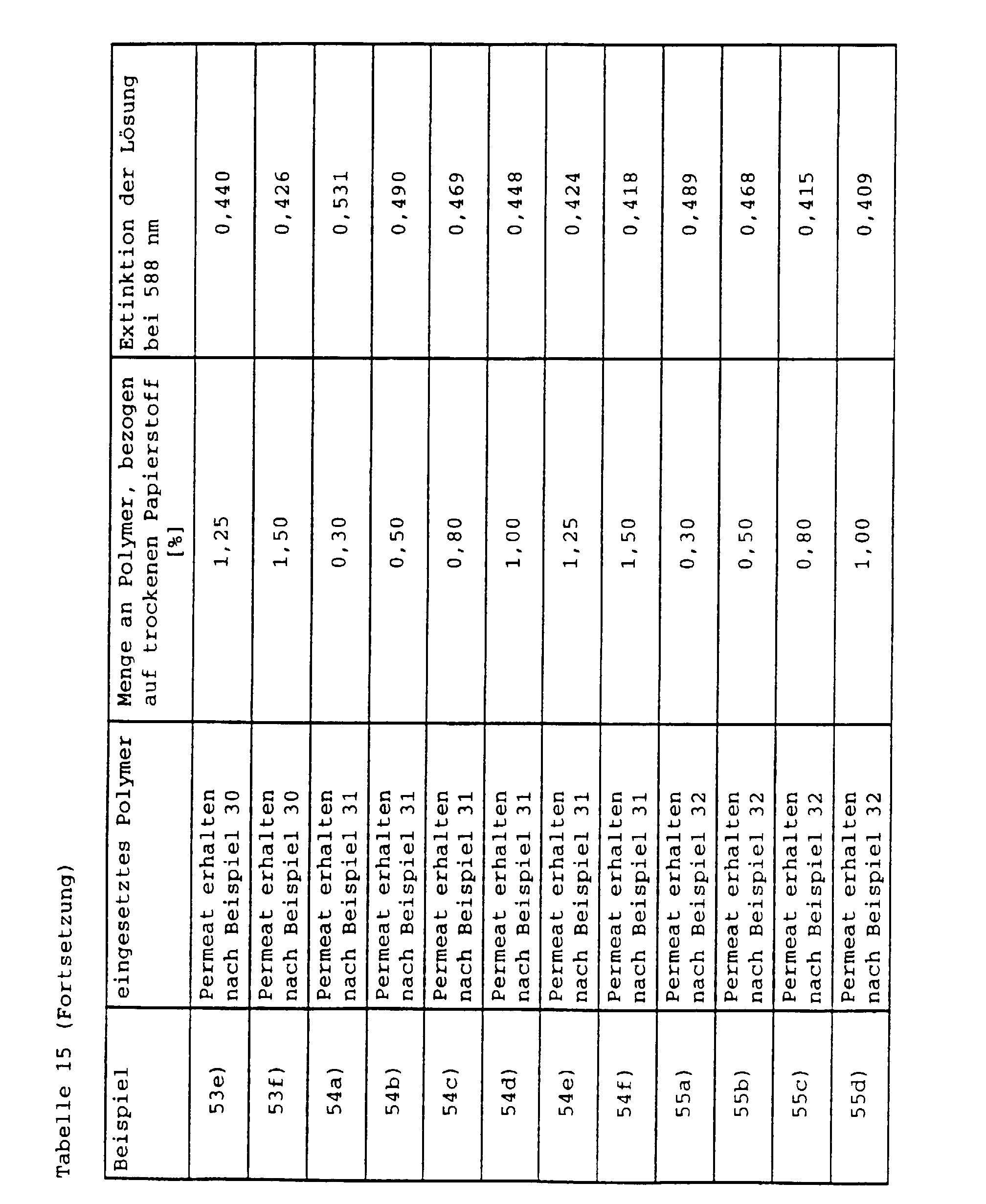

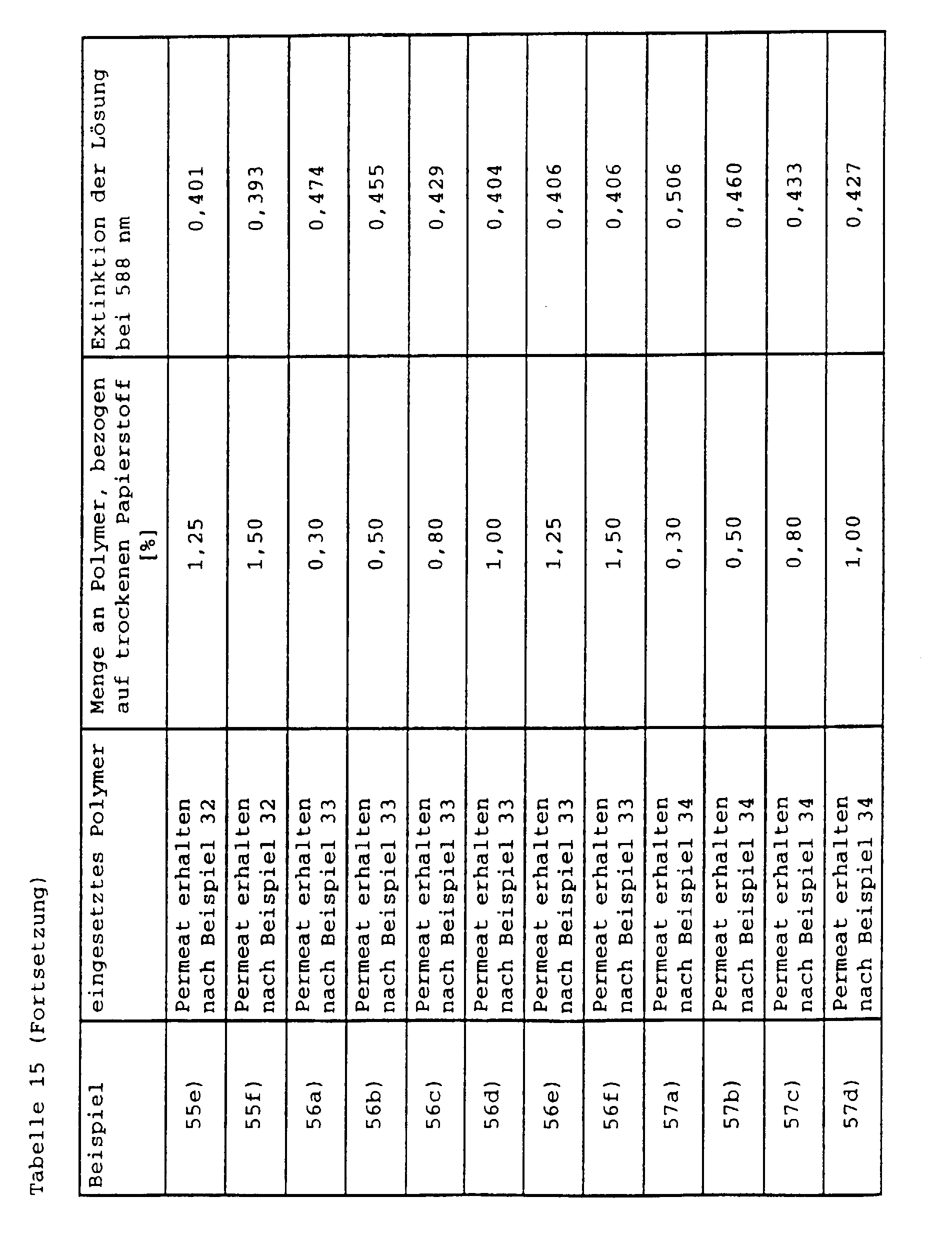

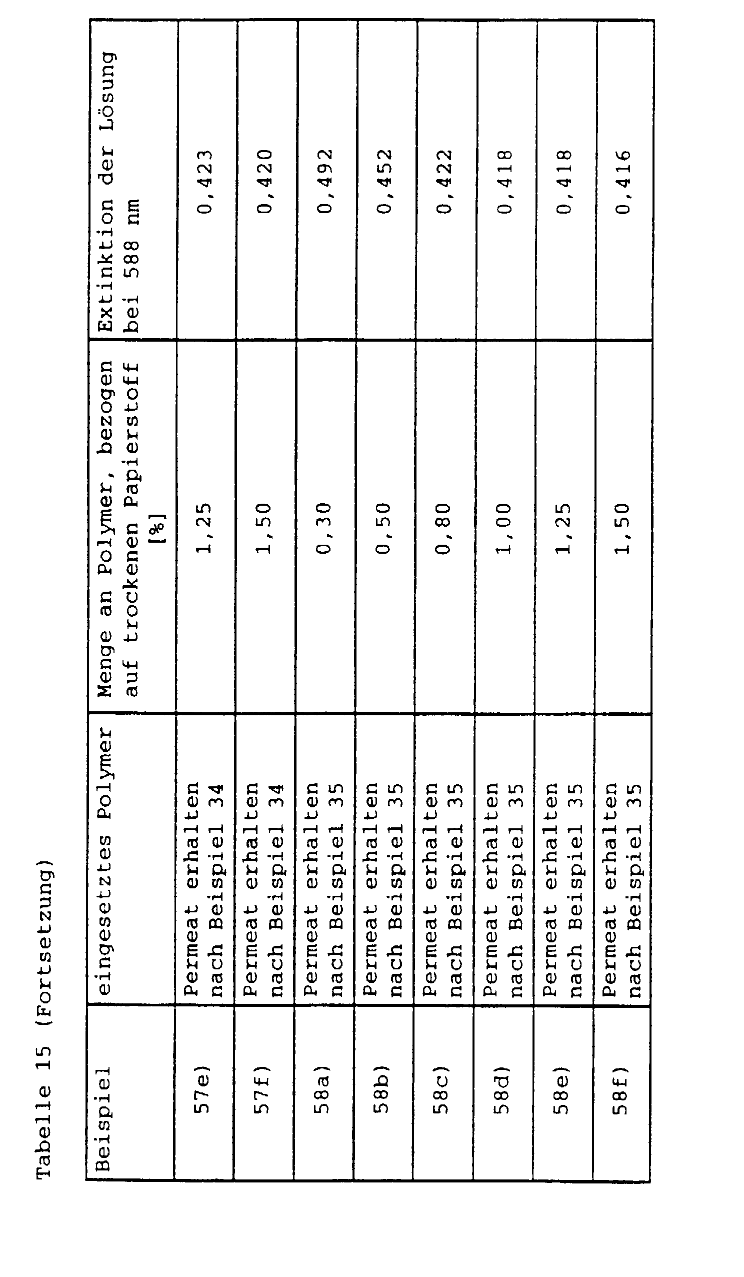

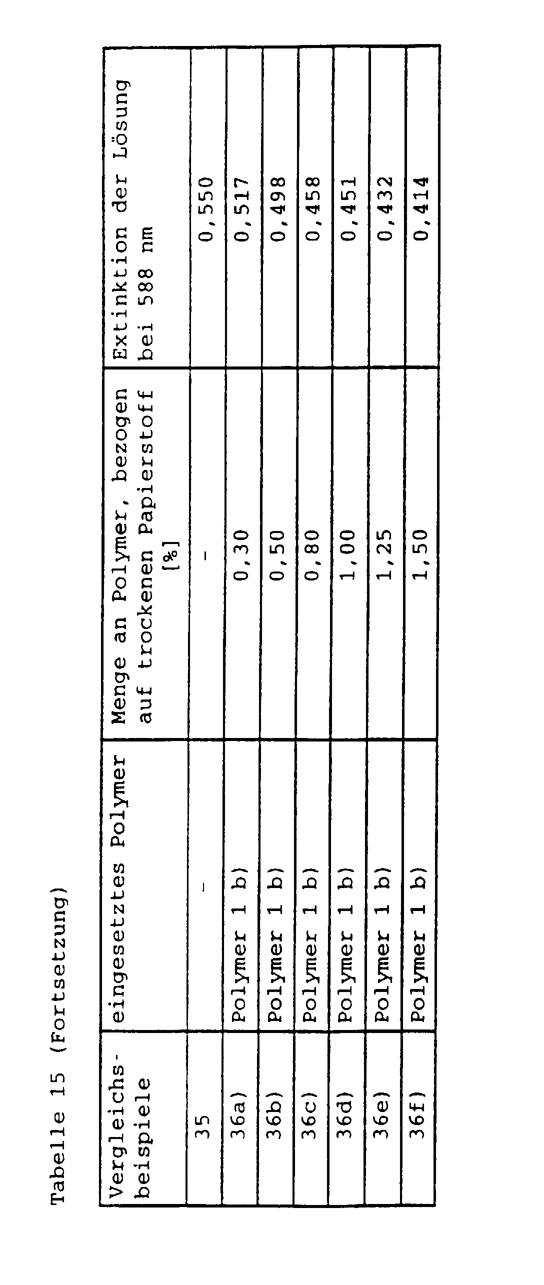

### Beispiel 59 (Polymere 3, 3a, 3b)

10,0 kg einer 10 %igen wäßrigen Lösung des handelsüblichen Polyamins Retaminol® SH (= Polymer 3) der Fa. Bayer AG mit einer Ladungsdichte von 8,1 meq/g (pH = 7) werden bei einem pH-Wert von 8,5 und unter Zusatz von 40 kg Wasser an einer Ultrafiltrationsmembran der Fa. A/G-Technology mit einer Trenngrenze von 100 000 bis zu einer Leitfähigkeit von 0,03 mS/cm diafiltriert. Das Retentat (Polymer 3a) wird nach Aufkonzentration als 3,8 %ige wäßrige Lösung erhalten. Insgesamt werden 71 Gew.-% des gelösten Ausgangsprodukts als Permeat (Polymer 3b) abgetrennt.

### Beispiel 60 (Polymere 4, 4a)

3,4 kg einer 8 %igen wäßrigen Lösung des handelsüblichen Polyamins Cartaretin® I (= Polymer 4) der Fa. Clariant mit einer Ladungsdichte von 4,9 meq/g bei pH = 7 werden bei einem pH-Wert von 7,1 mit insgesamt 17 kg Wasser an einer Ultrafiltrationsmembran der Fa. A/G-Technology mit einer Trenngrenze von 100 000 bis zu einer Leitfähigkeit von 0,09 mS/cm diafiltriert. Das Retentat (Fraktion > 100 000, Polymer 4a) wird auf eine Konzentration von 8,2 % eingeengt. Als Permeat (Fraktion < 100 000) werden 71 Gew.-% des gelösten Ausgangsprodukts abgetrennt.

### Beispiel 61 (Polymere 5, 5a, 5b)

5,0 kg einer 5 %igen wäßrigen Lösung des Polyamins Catiofast SF (= Polymer 5) der Fa. BASF mit einer Ladungsdichte von 12,3 meq/g bei pH = 7 werden bei einem pH-Wert von 12,1 mit insgesamt 15 kg Wasser an einer Ultrafiltrationsmembran der Fa. A/G-Technology mit einer Trenngrenze von 100 000 bis zu einer Leitfähigkeit von 0,07 mS/cm diafiltriert. (Man trennt dadurch 40 Gew.-% des gelösten Ausgangsprodukts als Permeat - Polymer 5b - ab.) Nach Einstellung mit Ameisensäure auf einen pH-Wert von 7,5 wird das Retentat - Polymer 5a - auf eine 14,1 %ige wäßrige Lösung aufkonzentriert.

### Beispiel 62 (Polymere 6, 6a, 6b)

18,1 kg einer 10 %igen wäßrigen Lösung des Polyamins Catiofast PL (= Polymer 6) der Fa. BASF mit einer Ladungsdichte von 12,6 meq/g werden bei einem pH-Wert von 11,3 mit insgesamt 24 kg Wasser an einer Ultrafiltrationsmembran der Fa. A/G-Technology mit einer Trenngrenze von 100 000 bis zu einer Leitfähigkeit von 0,16 mS/cm diafiltriert. Nach Abtrennen von 47 Gew.-% des gelösten Ausgangsprodukts als Permeat - Polymer 6b - isoliert man das Retentat (Fraktion > 100 000, Polymer 6a). Nach Aufkonzentrieren und Zugabe von Ameisensäure bis zu einem pH von 7,5 liegt eine 27,6 %ige wäßrige Lösung vor.

### Beispiel 63 und Vergleichsbeispiel 37

Aus 100 Teilen unbedrucktem Zeitungspapier mit einem Füllstoffgehalt von ca. 10 % und 10 Teilen China Clay (Type XI der Firma ECC) wurde eine Pulpe der Stoffdichte 5 g/l und einem Mahlgrad von 58°SR (Schopper-Riegler) hergestellt. Der pH-Wert der Pulpe betrug 7,6. Der Papierstoff wurde in mehrere Proben geteilt, die dann mit den in Tabelle 16 angegebenen Mengen an Polymer versetzt wurden. Von jeder Probe wurde die Entwässerungszeit für jeweils 600 ml Filtrat im Schopper-Riegler-Testgerät bestimmt.

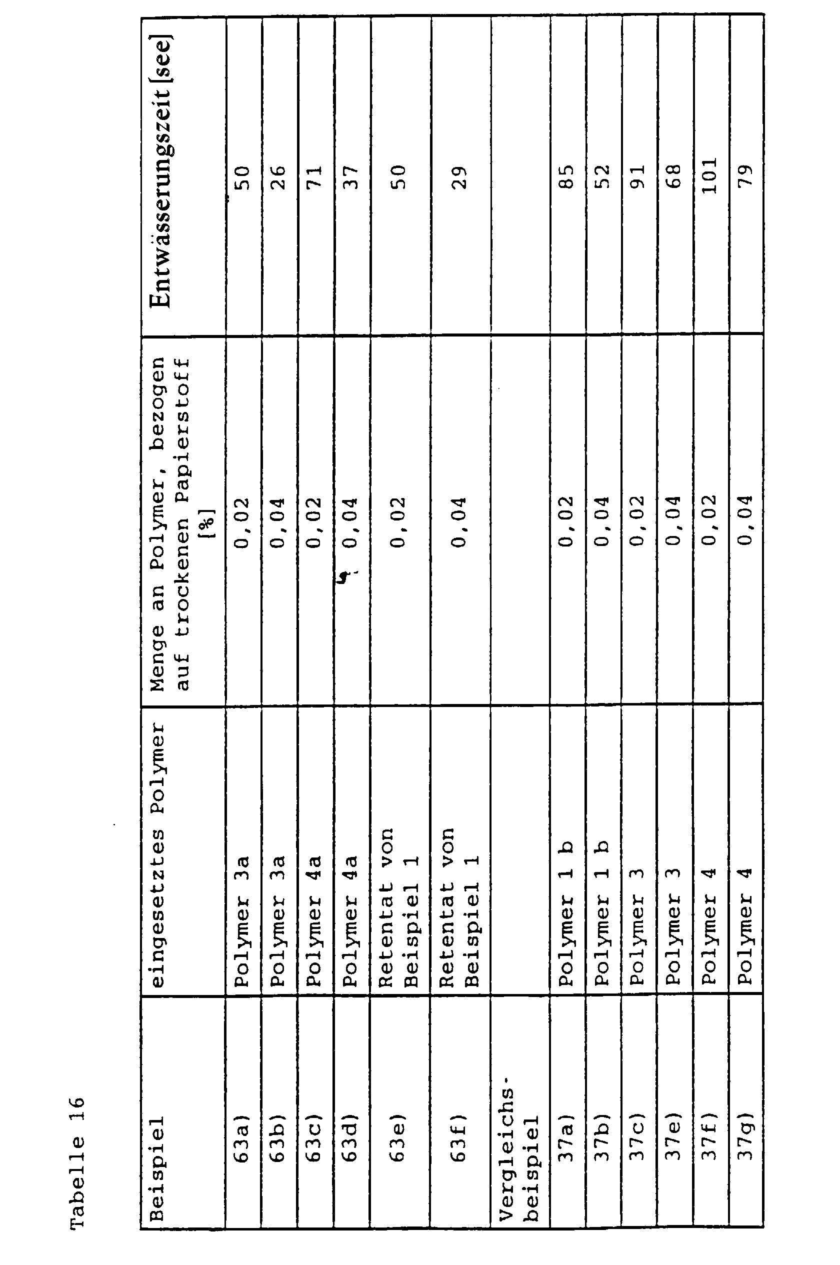

### Beispiel 64 und Vergleichsbeispiel 38

Aus 100 Teilen bedrucktem Zeitungspapier wurde eine Pulpe mit einer Stoffdichte von 5 g/l und einem Mahlgrad von 50°SR hergestellt, Der pH-Wert der Pulpe betrug 7,6. Zu Proben dieses Papierstoffs wurden die in Tabelle 17 angegebenen Mengen an Polymeren zugesetzt und im Schopper-Riegler-Testgerät die Entwässerungszeit für jeweils 600 ml Filtrat bestimmt.

**Tabelle 17**

| Beispiel | eingesetztes Polymer | Menge an Polymer, bezogen auf trockenen Papierstoff [%] | Entwässerungszeit [sec.] |
|---|---|---|---|
| 64a) | Polymer 3a | 0,02 | 130 |
| 64b) | Polymer 3a | 0,04 | 121 |
| 64c) | Polymer 4a | 0,02 | 122 |
| 64d) | Polymer 4a | 0,04 | 73 |
| 64e) | Retentat von Beispiel 1 | 0,02 | 90 |
| 64f) | Retentat von Beispiel 1 | 0,04 | 47 |

| Vergieichsbeispiel | | | |
|---|---|---|---|
| 38a) | Polymer 1 b | 0,02 | 114 |
| 38b) | Polymer 1 b | 0,04 | 93 |
| 38c) | Polymer 3 | 0,02 | 130 |
| 38d) | Polymer 3 | 0,04 | 121 |
| 38e) | Polymer 4 | 0,02 | 138 |
| 38f) | Polymer 4 | 0,04 | 117 |

### Beispiel 65 und Vergleichsbeispiel 39

Aus 50 Teilen gebleichtem Kiefernsulfatzellstoff, 50 Teilen gebleichtem Buchensulfitzellstoff und 30 Teilen einer natürlichen Kreide (Typ DX1 der Firma Omya) wurde eine Pulpe mit einer Stoffdichte von 5 g/l hergestellt. Der pH-Wert der Pulpe betrug 7,2. Proben des Papierstoffs wurden mit den in Tabelle 18 angegebenen Mengen an Polymer gemischt und im Schopper-Riegler-Testgerät die Entwässerungszeiten für 600 ml Filtrat bestimmt.

**Tabelle 18**

| Beispiel | eingesetztes Polymer | Menge an Polymer. bezogen auf trockenen Papierstoff[%] | Entwässerungszeit [sec.] |
|---|---|---|---|
| 65a) | Polymer 3a | 0,02 | 37 |
| 65b) | Polymer 3a | 0,04 | 28 |
| 65c) | Polymer 4a | 0,02 | 44 |
| 65d) | Polymer 4a | 0,04 | 29 |
| 65e) | Retentat von Beispiel 1 | 0,02 | 35 |
| 65f) | Retentat von Beispiel 1 | 0,04 | 19 |

| Vergleichsbeispiel | | | |
|---|---|---|---|
| 39a) | Polymer 1 b | 0,02 | 41 |
| 39b) | Polymer 1 b | 0,04 | 36 |
| 39c) | Polymer 3 | 0,02 | 54 |
| 39d) | Polymer 3 | 0,04 | 47 |
| 39e) | Polymer 4 | 0,02 | 49 |
| 39f) | Polymer 4 | 0,04 | 47 |

### Beispiel 66-69 und Vergleichsbeispiele 40-43

Eine wäßrige Faseraufschlämmung aus TMP (thermomechanische Pulpe) mit einer Stoffkonzentration von 2 % wurde in gleiche Anteile geteilt und jeweils mit einer wäßrigen Lösung aus Holzextrakt, als Störstoff versetzt. Zu Proben dieser Pulpe gab man jeweils die in Tabelle 19 angegebene Mengen an zu prüfendem Polymer sowie zusätzlich noch jeweils die gleiche Menge eines kationischen Polyacrylamids als Flockungsmittel. Nach Durchmischen und Filtration des geflockten Papierstoffs wird die Extinktion des alkalischen Filtrats bei 340 nm bestimmt.

**Tabelle 19**

| Beispiel | eingesetztes Polymer | Menge an Polymer, bezogen auf trockenen Papierstoff [%] | Extinktion des Filtrats [gemessen bei 340 nm] |
|---|---|---|---|
| 66a) | Polymer 5a | 0,30 | 0,467 |
| 66b) | Polymer 5a | 0,50 | 0,403 |
| 66c) | Polymer 5a | 0,80 | 0,374 |
| 66d) | Polymer 5a | 1,00 | 0,321 |
| 66e) | Polymer 5a | 1,25 | 0,303 |
| 66f) | Polymer 5a | 1,50 | 0,263 |
| 67a) | Polymer 6a | 0,30 | 0,425 |
| 67b) | Polymer 6a | 0,50 | 0,336 |
| 67c) | Polymer 6a | 0,80 | 0,312 |
| 67d) | Polymer 6a | 1,00 | 0,282 |
| 67e) | Polymer 6a | 1,25 | 0,246 |
| 67f) | Polymer 6a | 1,50 | 0,235 |
| 68a) | Polymer 3a | 0,30 | 0,480 |
| 68b) | Polymer 3a | 0,50 | 0,435 |
| 68c) | Polymer 3a | 0,80 | 0,312 |
| 68d) | Polymer 3a | 1,00 | 0,393 |
| 68e) | Polymer 3a | 1,25 | 0,344 |
| 68f) | Polymer 3a | 1,50 | 0,356 |
| 69a) | Polymer 4a | 0,30 | 0,479 |
| 69b) | Polymer 4a | 0,50 | 0,444 |
| 69c) | Polymer 4a | 0,80 | 0,404 |
| 69d) | Polymer 4a | 1,00 | 0,396 |
| 69e) | Polymer 4a | 1,25 | 0,362 |
| 69f) | Polymer 4a | 1,50 | 0,344 |

| Vergleichsbeispiel | | | |
|---|---|---|---|
| 40a) | Polymer 5 | 0,30 | 0,483 |
| 40b) | Polymer 5 | 0,50 | 0,422 |
| 40c) | Polymer 5 | 0,80 | 0,388 |
| 40d) | Polymer 5 | 1,00 | 0,361 |
| 40e) | Polymer 5 | 1,25 | 0,350 |
| 40f) | Polymer 5 | 1,50 | 0,316 |
| 41a) | Polymer 6 | 0,30 | 0,429 |
| 41b) | Polymer 6 | 0,50 | 0,383 |
| 41c) | Polymer 6 | 0,80 | 0,347 |
| 41d) | Polymer 6 | 1,00 | 0,336 |
| 41e) | Polymer 6 | 1,25 | 0,323 |
| 41f) | Polymer 6 | 1,50 | 0,271 |
| 42a) | Polymer 3 | 0,30 | 0,479 |
| 42b) | Polymer 3 | 0,50 | 0,460 |
| 42c) | Polymer 3 | 0,80 | 0,451 |
| 42d) | Polymer 3 | 1,00 | 0,438 |
| 42e) | Polymer 3 | 1,25 | 0,404 |
| 42f) | Polymer 3 | 1,50 | 0,398 |
| 43a) | Polymer 4 | 0,30 | 0,529 |
| 43b) | Polymer 4 | 0,50 | 0,491 |
| 43c) | Polymer 4 | 0,80 | 0,471 |
| 43d) | Polymer 4 | 1,00 | 0,464 |
| 43e) | Polymer 4 | 1,25 | 0,440 |
| 43f) | Polymer 4 | 1,50 | 0,420 |

## Patentansprüche

1. Verfahren zur Herstellung von wasserlöslichen, Aminogruppen enthaltenden Kondensaten und Additionsprodukten mit erhöhter Entwässerungs- und Retentionswirkung bei der Herstellung von Papier, dadurch gekennzeichnet, daß man wäßrige Lösungen von Kondensaten oder Additionsprodukten aus der Gruppe der
- Reaktionsprodukte von Alkylendiaminen, Polyalkylenpolyaminen, mit Ethylenimin gepfropften Polyamidoaminen sowie deren Mischungen mit mindestens zwei funktionelle Gruppen aufweisenden Vernetzern,
- Reaktionsprodukte von Michaeladditionsprodukten aus Polyalkylenpolyaminen, Polyamidoaminen, mit Ethylenimin gepfropften Polyamidoaminen sowie deren Mischungen und monoethylenisch ungesättigten Carbonsäuren sowie deren Salzen, Estern, Amiden oder Nitrilen mit mindestens bifunktionellen Vernetzern,
- amidierten Polyethylenimine, die erhältlich sind durch Reaktion von Polyethyleniminen mit einbasischen Carbonsäuren oder ihren Estern, Anhydriden, Säurechloriden oder Säureamiden und gegebenenfalls Umsetzung der amidierten Polyethylenimine mit mindestens zwei funktionelle Gruppen aufweisenden Vernetzern,
- Polyethylenimine, quaternisierten Polyethylenimine, phosphonomethylierten Polyethylenimine, alkoxylierten Polyethylenimine und/oder durch Streckerreaktion carboxymethylierten Polyethylenimine und
- vernetzten alkoxylierten Polyethylenimine, vernetzten, quaternisierten Polyethylenimine, vernetzten, phosphonomethylierten Polyethylenimine und/oder vernetzten, durch Streckerreaktion carboxymethylierten Polyethylenimine
einer Ultrafiltration an Membranen unterwirft, wobei man 5 bis 95 Gew.-% der Kondensate oder Additionsprodukte als Permeat abtrennt und die wasserlöslichen, Aminogruppen enthaltenden Kondensate oder Additionsprodukte mit verbesserter Wirksamkeit gegebenenfalls aus dem Retentat isoliert.

2. Verfahren nach Anspruch 1, dadurch gekennzeichnet, daß man 20 bis 90 Gew.-%, bezogen auf die eingesetzten Polymeren, als Permeat abtrennt.

3. Verfahren nach Anspruch 1 oder 2, dadurch gekennzeichnet, daß man die Ultrafiltration an Membranen mit einem mittleren Porendurchmesser von 0,001 bis 10 µm oder an Membranen mit einer Trenngrenze für Polymere mit Molmassen von 1000 bis 10 Millionen durchführt.

4. Verfahren nach Anspruch 1 oder 2, dadurch gekennzeichnet, daß man die Ultrafiltration an Membranen mit einer Trenngrenze für Polymere mit Molmassen von mindestens 1500 bis 500000 durchführt.

5. Verfahren nach Anspruch 1 oder 2, dadurch gekennzeichnet, daß man die Ultrafiltration an Membranen mit einer Trenngrenze von mindestens 100 000 durchführt.

6. Verfahren nach einem der Ansprüche 1 bis 5, dadurch gekennzeichnet, daß man die Membranen in Form von Rohren, Hohlfasern, Plattengeräten oder Spiral-Wickel-Modulen und die Aminogruppen enthaltenden Kondensate oder Additionsprodukte als wäßrige Lösungen einsetzt.

7. Verfahren nach den Ansprüchen 1 bis 6, dadurch gekennzeichnet, daß man die als Permeat abgetrennten Anteile an Kondensaten oder Additionsprodukten in den Herstellungsprozeß der bei der Ultrafiltration als Ausgangsprodukte eingesetzten wasserlöslichen, Aminogruppen enthaltenden Kondensate oder Additionsprodukte zurückführt.

8. Verfahren nach Anspruch 7, dadurch gekennzeichnet, daß man die als Permeat abgetrennten Anteile an Kondensaten oder Additionsprodukten
- mit Ethylenimin pfropft und/oder
- mit mindestens zwei funktionelle Gruppen aufweisenden Vernetzern zu wasserlöslichen, Aminogruppen enthaltenden Kondensaten oder Additionsprodukten umsetzt und
die so erhältlichen Lösungen erneut der Ultrafiltration an Membranen unterwirft, wobei man 5 bis 95 Gew.-% der Kondensate oder Additionsprodukte aus den Lösungen als Permeat abtrennt.

9. Verfahren nach einem der Ansprüche 1 bis 8, dadurch gekennzeichnet, daß man als wasserläsliche Aminogruppen enthaltende Kondensate die Reaktionsprodukte einsetzt, die durch Pfropfen von Polyamidoaminen mit Ethylenimin und anschließende Reaktion der gepfropften Produkte mit Bischlorhydrinethern oder Bisglycidylethern von Polyalkylenglykolen als Vernetzer erhältlich sind.

10. Verfahren nach Anspruch 9, dadurch gekennzeichnet, daß man als wasserlösliche Aminogruppen enthaltende Kondensate die Reaktionsprodukte einsetzt, die durch Vernetzung von mit Ethylenimin gepfropften Polyamidoaminen mit Bisglycidylethern von Polyalkylenglykolen, die 8 bis 100 Ethylenoxid- und/oder Propylenoxid-Einheiten enthalten, erhältlich sind.

11. Verfahren nach den Ansprüchen 1 bis 10, dadurch gekennzeichnet, daß man halogenfreie Vernetzer einsetzt, die ausgewählt sind aus der Gruppe bestehend aus
(1) Ethylencarbonat, Propylencarbonat und/oder Harnstoff,
(2) monoethylenisch ungesättigten Carbonsäuren und deren Estern, Amiden und Anhydriden, mindestens zweibasischen gesättigten Carbonsäuren oder Polycarbonsäuren sowie den jeweils davon abgeleiteten Estern, Amiden und Anhydriden,
(3) Umsetzungsprodukten von Polyetherdiaminen, Alkylendiaminen, Polyalkylenpolyaminen, Alkylenglykolen, Polyalkylenglykolen oder deren Gemischen mit monoethylenisch ungesättigten Carbonsäuren, Estern, Amiden oder Anhydriden monoethylenisch ungesättigter Carbonsäuren, wobei die Umsetzungsprodukte mindestens zwei ethylenisch ungesättigte Doppelbindungen, Carbonsäureamid-, Carboxyl- oder Estergruppen als funktionelle Gruppen aufweisen,
(4) mindestens zwei Aziridinogruppen enthaltenden Umsetzungsprodukten von Dicarbonsäureestern mit Ethylenimin
sowie Mischungen der genannten Vernetzer.

12. Verwendung der nach den Ansprüchen 1 bis 11 erhältlichen hochmolekularen Anteile an Polymeren als Retentions-, Entwässerungs- und Fixiermittel bei der Herstellung von Papier, als Promotor bei der Leimung von Papier mit Alkyldiketenen, als Flockungsmittel für Klärschlämme, als Haftvermittler bei der Herstellung von Verbundfolien, als Additiv in Haar- und Hautpflegemitteln und als Mittel zur Immobilisierung von anionischen Wirkstoffen.

## Claims

1. A process for the preparation of water-soluble, amino-containing condensates and adducts having a greater drainage and retention effect in papermaking, wherein aqueous solutions of condensates or adducts selected from the group consisting of
- reaction products of alkylenediamines, polyalkylenepolyamines, ethyleneimine-grafted polyamidoamines and mixtures thereof with crosslinking agents having at least two functional groups,
- reaction products of Michael adducts of polyalkylene polyamines, polyamidoamines, ethyleneimine-grafted polyamidoamines and mixtures thereof and monoethylenically unsaturated carboxylic acids and salts, esters, amides or nitriles thereof with at least bifunctional crosslinking agents,
- amidated polyethyleneimines which are obtainable by reaction of polyethyleneimines with monobasic carboxylic acids or their esters, anhydrides, acid chlorides or acid amides and, if required, reaction of the amidated polyethyleneimines with crosslinking agents having at least two functional groups,
- polyethyleneimines, quaternized polyethyleneimines, phosphonomethylated polyethyleneimines, alkoxylated polyethyleneimines and/or polyethyleneimines carboxymethylated by a Strecker reaction and
- crosslinked alkoxylated polyethyleneimines, crosslinked, quaternized polyethyleneimines, crosslinked, phosphonomethylated polyethyleneimines and/or crosslinked polyethyleneimines carboxymethylated by a Strecker reaction
are subjected to an ultrafiltration through membranes, from 5 to 95% by weight of the condensates or adducts being separated off as permeate and the water-soluble, amino-containing condensates or adducts having improved efficiency, if required, isolated from the retentate.

2. A process as claimed in claim 1, wherein from 20 to 90 % by weight, based on the polymers used, are separated off as permeate.

3. A process as claimed in claim 1 or 2, wherein the ultrafiltration is carried out through membranes having a mean pore diameter of from 0.001 to 10 mm or through membranes having a cut-off for polymers with molar masses of from 1000 to 10 million.

4. A process as claimed in claim 1 or 2, wherein the ultrafiltration is carried out through membranes having a cut-off for polymers with molar masses of from at least 1500 to 500,000.

5. A process as claimed in claim 1 or 2, wherein the ultrafiltration is carried out through membranes having a cut-off of at least 100,000.

6. A process as claimed in any of claims 1 to 5, wherein the membranes are used in the form of tubes, hollow fibers, plate-type apparatuses or spiral wound modules and the amino-containing condensates or adducts are used in the form of aqueous solutions.

7. A process as claimed in any of claims 1 to 6, wherein those fractions of condensates or adducts which are separated off as permeate are recycled to the preparation process for the water-soluble, amino-containing condensates or adducts used as starting materials in the ultrafiltration.

8. A process as claimed in claim 7, wherein those fractions of condensates or adducts which are separated off as permeate
- are grafted with ethyleneimine or
- are reacted with crosslinking agents having at least two functional groups to give water-soluble, amino-containing condensates or adducts and
the solutions thus obtainable are then subjected to the ultrafiltration through membranes, from 5 to 95 % by weight of the condensates or adducts being separated off from the solutions as permeate.

9. A process as claimed in any of claims 1 to 8, wherein the water-soluble amino-containing condensates used are the reaction products which are obtainable by grafting polyamidoamines with ethyleneimine and then reacting the grafted products with bischlorohydrin ethers or bisglycidyl ethers of polyalkylene glycols as crosslinking agents.

10. A process as claimed in claim 9, wherein the water-soluble amino-containing condensates used are the reaction products which are obtainable by crosslinking ethyleneimine-grafted polyamidoamines with bisglyidyl ethers of polyalkylene glycols which contain from 8 to 100 ethylene oxide and/or propylene oxide units.

11. A process as claimed in any of claims 1 to 10, wherein the halogen-free crosslinking agent used is selected from the group consisting of
(1) ethylene carbonate, propylene carbonate or urea,
(2) monoethylenically unsaturated carboxylic acids and esters, amides and anhydrides thereof, at least dibasic saturated carboxylic acids or polycarboxylic acids and the esters, amides and anhydrides derived from each of them,
(3) reaction products of polyetherdiamines, alkylenediamines, polyalkylenepolyamines, alkylene glycols, polyalkylene glycols or mixtures thereof with monoethylenically unsaturated carboxylic acids, esters, amides or anhydrides of monoethylenically unsaturated carboxylic acids, the reaction products having at least two ethylenically unsaturated double bonds, and carboxamido, carboxyl or ester groups as functional groups,
(4) reaction products of dicarboxylic esters with ethyleneimine, which reaction products contain at least two aziridino groups,
and mixtures of the stated crosslinking agents.

12. The use of the high molecular weight polymer fractions obtainable as claimed in any of claims 1 to 11 as retention aids, drainage aids and fixing compositions in papermaking, as promoters in the sizing of paper with alkyldiketenes, as flocculants for sewage sludges, as adhesion promoters in the production of laminated films, as additives in hair and skincare compositions and as compositions for immobilizing anionic active ingredients.

## Revendications

1. Procédé de préparation de condensats et de produits d'addition hydrosolubles et contenant des groupements amino, ayant des propriétés de rétention et de dessiccation améliorées lors de la fabrication de papier, caractérisé en ce que l'on soumet à une ultrafiltration sur membranes des solutions aqueuses de condensats ou de produits d'addition choisis dans le groupe formé par
- les produits de la réaction d'alkylènediamines, de polyalkylène-polyamines, de polyamidoamines greffées à l'éthylène-imine ainsi que leurs mélanges, avec des réticulants ayant au moins deux groupements fonctionnels,
- les produits de la réaction de produits d'addition de Michael à base de polyalkylènepolyamines, de polyamidoamines, de polyamidoamines greffées à l'éthylène-imine ainsi que leurs mélanges, et d'acides carboxyliques à insaturation monoéthylénique ainsi que leurs sels, esters, amides ou nitriles, avec des réticulants au moins bifonctionnels,
- des polyéthylène-imines amidifiées pouvant être obtenues par réaction de polyéthylène-imines avec des acides carboxyliques monoacides ou leurs esters, anhydrides, chlorures d'acides ou carboxamides et éventuellement réaction de la polyéthylène-imine amidifiée avec des réticulants ayant au moins deux groupements fonctionnels,
- des polyéthylène-imines, des polyéthylène-imines quaternisées, des polyéthylène-imines phosphonométhylées, des polyéthylène-imines alcoxylées et/ou des polyéthylène-imines carboxyméthylées par une réaction de Strecker et
- des polyéthylène-imines alcoxylées et réticulées, des polyéthylène-imines quaternisées et réticulées, des polyéthylène-imines phosphonométhylées et réticulées et/ou des polyéthylène-imines carboxyméthylées par une réaction de Strecker et réticulées,
où 5-95% en poids des condensats ou des produits d'addition sont séparés en tant en tant que perméat, et on isole éventuellement du rétentat les condensats ou produits d'addition hydrosolubles contenant des groupements amine ayant des propriétés améliorées.

2. Procédé selon la revendication 1, caractérisé en ce que l'on sépare 20-90% en poids, par rapport aux polymères utilisés, en tant que perméat.

3. Procédé selon la revendication 1 ou 2, caractérisé en ce que l'on mène l'ultrafiltration sur des membranes ayant un diamètre de pores moyen de 0,001-10 µm ou sur des membranes ayant une limite de séparation pour les polymères de masse molaire de 1000 à 10 millions.

4. Procédé selon la revendication 1 ou 2, caractérisé en ce que l'on mène l'ultrafiltration sur des membranes ayant une limite de séparation pour les polymères de masse molaire d'au moins 1500 à 500 000.

5. Procédé selon la revendication 1 ou 2, caractérisé en ce que l'on mène l'ultrafiltration sur des membranes ayant une limite de séparation d'au moins 100 000.

6. Procédé selon l'une quelconque des revendications 1 à 5, caractérisé en ce que l'on utilise des membranes sous forme de tubes, de fibres creuses, de dispositifs à plaques ou de modules de bobines spiralées et les condensats ou les produits d'addition contenant des groupements amino sous forme de solutions aqueuses.

7. Procédé selon l'une quelconque des revendications 1 à 6, caractérisé en ce que l'on recycle les fractions séparées en tant que perméat des condensats ou produits d'addition dans le processus de fabrication des produits d'addition ou des condensats hydrosolubles et contenant des groupements amino utilisés en tant que produits de départ pour l'ultrafiltration.

8. Procédé selon la revendication 7, caractérisé en ce que les fractions séparées en tant que perméat des condensats ou produits d'addition
- sont greffées avec de l'éthylène-imine et/ou
- sont mises à réagir avec des réticulants comprenant au moins deux groupements fonctionnels pour donner des condensats ou des produits d'addition hydrosolubles et comportant des groupements amino et
les solutions ainsi obtenues sont soumises à l'ultrafiltration sur membranes, où 5-95% en poids des condensats ou des produits d'addition sont séparés des solutions en tant que perméat.

9. Procédé selon l'une quelconque des revendications 1 à 8, caractérisé en ce que l'on utilise en tant que condensats hydrosolubles contenant des groupements amino, les produits réactionnels pouvant être obtenus par greffage de polyamidoamines avec de l'éthylène-imine suivi de la réaction des produits greffés avec des bischlorhydrineéthers ou des bisglycidyléthers de polyalkylèneglycols en tant que réticulants.

10. Procédé selon la revendication 9, caractérisé en ce que l'on utilise en tant que condensats hydrosolubles contenant des groupements amino, les produits réactionnels pouvant être obtenus par réticulation de polyamidoamines greffées avec de l'éthylène-imine avec des bisglycidyléthers de polyalkylèneglycols contenant 8-100 unités d'oxyde d'éthylène et/ou d'oxyde de propylène.

11. Procédé selon l'une quelconque des revendications 1 à 10, caractérisé en ce que l'on utilise des réticulants exempts d'halogènes, choisis dans le groupe formé par
(1) le carbonate d'éthylène, le carbonate de propylène et/ou l'urée,
(2) les acides carboxyliques à insaturation monoéthylénique et leurs esters, amides et anhydrides, les acides carboxyliques saturés ayant au moins deux acidités ou les acides polycarboxyliques ainsi que leurs dérivés esters, amides et anhydrides,
(3) les produits de la réaction de polyétherdiamines, d'alkylènediamines, de polyalkylènepolyamines, d'alkylène-glycols, de polyalkyléneglycols ou de leurs mélanges avec des acides carboxyliques à insaturation monoéthylénique, des esters, des amides ou des anhydrides d'acides carboxyliques à insaturation monoéthylénique, où les produits réactionnels présentent au moins deux doubles liaisons à insaturation éthylénique, des groupements carboxamido, carboxyle ou esters en tant que groupements fonctionnels,
(4) des produits de la réaction d'esters d'acides dicarboxyliques avec de l'éthylène-imine, comprenant au moins deux groupements aziridino,
ainsi que des mélanges des réticulants cités.

12. Utilisation des fractions à haut poids moléculaire de polymères pouvant être obtenus selon l'une quelconque des revendications 1 à 11, en tant qu'agent de rétention, de dessiccation et de fixation lors de la fabrication du papier, en tant que promoteur lors de l'enduction de papier avec des alkyldicétènes, en tant qu'agent de floculation pour des eaux résiduaires, en tant qu'agent d'adhésion lors de la préparation de feuilles composites, en tant qu'additif pour des agents de soin des cheveux et de la peau et en tant qu'agent d'immobilisation pour des matières anioniques.
